# EUROPÄISCHE PATENTSCHRIFT

(11) **EP 1 967 523 B1**
(45) Veröffentlichungstag und Bekanntmachung des Hinweises auf die Patenterteilung: **11.08.2010**
(21) Anmeldenummer: 08008232.4
(22) Anmeldetag: 27.06.2003
(51) Int. Cl.: C07H 17/00, A61K 31/7042, A61P 3/10

(54) **Neue Thiophenglycosidderivate, Verfahren zu deren Herstellung, diese Verbindungen enthaltende Arzneimittel und deren Verwendung**
New thiophen glycoside derivatives, method for their manufacture, medicines containing these compounds and their application
Nouveaux dérivés de glycoside de thiofène, son procédé de fabrication, médicament comportant ces liaisons et son utilisation

(30) Priorität: 11.07.2002 DE 10231370
(43) Veröffentlichungstag der Anmeldung: 10.09.2008
(62) Teilanmeldung aus: 03763662.8
(73) Patentinhaber: Sanofi-Aventis Deutschland GmbH, 65929 Frankfurt am Main (DE)
(72) Erfinder: Glombik, Heiner, 65926 Frankfurt am Main (DE); Frick, Wendelin, 65926 Frankfurt am Main (DE); Heuer, Hubert, 65926 Frankfurt am Main (DE); Kramer, Werner, 65926 Frankfurt am Main (DE); Brummerhop, Harm, 65926 Frankfurt am Main (DE); Plettenburg, Oliver, 65926 Frankfurt am Main (DE)

(56) Entgegenhaltungen:
- EP-A- 1 213 296

## Beschreibung

Die Erfindung betrifft eine Zusammensetzung enthaltend substituierte Thiophenglycosidderivate und deren physiologisch verträgliche Salze.

Bekannt ist das Antirheumatikum Tenidap (ß-D-glucopyranosid-uronsäure, 5-[(Z)-[1-(aminocarbonyl)-5-chlor-1,2-dihydro-2-oxo-3H-indol-3-ylidenhydroxymethyl3-thienyl) (H.G. Fouda et al., CA: 1997:165448) sowie 3-Amino-2-benzoyl-5-glucopyranosylamino-thiophen-Verbindungen (J. Fuentes et al, Tetrahedron Asymmetry, 1998, 9, 2517-2532). EP1213296 beschreibt Pyrazolglykosidderivate, die den Natrium abhängigen Glukose Transporter 2 (SGLT-2) hemmen und sich zur Behandlung von Diabetes eigenen.

Der Erfindung lag die Aufgabe zugrunde, neue Verbindungen zur Verfügung zu stellen, mit denen eine Prävention und Behandlung von Diabetes Typ 1 und Typ 2 möglich ist.

Die Erfindung betrifft daher eine Zusammensetzung enthaltend eine Verbindung der Formel 1, 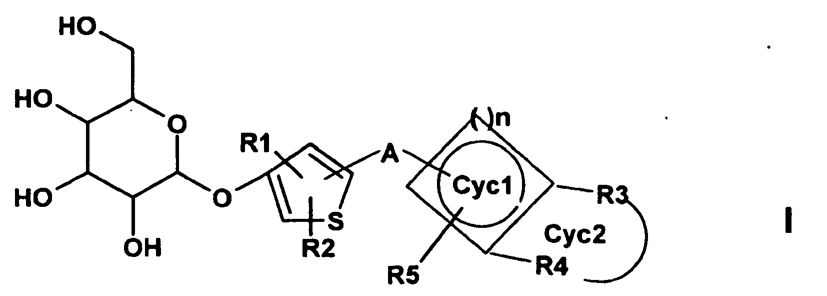 worin bedeuten
- R1, R2: Wasserstoff, F, Cl, Br, J, OH, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]₂, (C₁- (C₁-C₆)-Alkyl, (C₂-C₆)-Alkenyl, (C₂-C₆)-Alkinyl, (C₁-C₆)-Alkoxy, HO-(C₁-C₈)-Alkyl, (C₁-C₆)-Alkoxy-(C₁-C₆)-alkyl, Phenyl, Benzyl, (C₁-C₄)-Alkylcarbonyl, wobei in den Alkyl- und Alkoxyresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl, S-(CH₂)ₒ-Phenyl, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, (C₁-C₆)-Alkoxy, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, (C₁-C₆)-Alkoxy, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
- A: (C₀-C₁₅)-Alkandiyl, wobei ein oder mehrere C-Atome des Alkandiylrests unabhängig voneinander durch -O-, -(C=O)-, -CH=CH-, -C≡C-, -S-, -CH(OH)-, -CHF-, -CF₂-, -(S=O)-, -(SO₂)-, -N((C₁-C₆)-Alkyl)-, -N((C₁-C₆)-Alkyl-Phenyl)- oder -NH- ersetzt sein können;
- n: eine Zahl von 0 bis 4;
- Cyc1: ein 3 bis 7 gliedriger gesättigter, teilweise gesättigter oder ungesättigter Ring, wobei 1 C-Atom durch O oder S ersetzt sein kann;
- R3, R4, R5: Wasserstoff, F, Cl, Br, J, OH, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂, (C₁-C₈)-Alkyl, (C₂-C₆)-Alkenyl, (C₂-C₆)-Alkinyl, (C₁-C₁₂)-Alkoxy, HO-(C₁-C₆)-Alkyl, (C₁-C₆)-Alkoxy-(C₁-C₆)-alkyl, wobei in den Alkyl- und Alkoxyresten ein, mehrere, oder alle Wasserstoff(e) durch Fluor ersetzt sein können; SO₂-NH₂, SO₂NH(C₁-C₆)-Alkyl, SO₂N[(C₁-C₆)-Alkyl]₂, S-(C₁-C₆)-Alkyl, S-(CH₂)ₒ-Phenyl, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, (C₁-C₆)-Alkoxy, (C₁-C₆)- Alkyl, NH₂ substituiert sein kann; NH₂, NH-(C₁-C₆)-Alkyl, N((C₁-C₆)-Alkyl)₂, NH(C₁-C₇)-Acyl, Phenyl, (CH₂)₀- Phenyl, O-(CH₂)₀-Phenyl, wobei o = 0 - 6 sein kann, wobei der Phenylring ein bis 3-fach substituiert sein kann mit F, Cl, Br, J, OH, CF₃, NO₂, CN, OCF₃, (C₁-C₈)-Alkoxy, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂; oder
- R3 und R4: gemeinsam mit den sie tragenden C-Atomen einen 5 bis 7 gliedrigen, gesättigten, teilweise oder vollständig ungesättigten Ring Cyc2, wobei 1 oder 2 C-Atom(e) des Ringes auch durch N, O oder S ersetzt sein können und Cyc2 gegebenenfalls durch (C₁-C₆)-Alkyl, (C₂-C₅)-Alkenyl, (C₂-C₅)- Alkinyl, wobei jeweils eine CH₂-Gruppe durch O ersetzt sein kann, oder durch F, Cl, OH, CF₃, NO₂, CN, COO(C₁-C₄)-Alkyl, CONH₂, CONH(C₁- C₄)-Alkyl, OCF₃ substituiert sein kann und
- R5: Wasserstoff;
sowie deren pharmazeutisch verträglichen Salze.

Bevorzugt sind Verbindungen der Formel I, worin A mit dem Thienylring in 2 Position verknüpft ist.

Bevorzugt sind ferner Verbindungen der Formel I, worin bedeuten
- R1, R2: Wasserstoff, F, Cl, Br, J, OH, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]₂, (C₁- C₆)-Alkyl, (C₂-C₆)-Alkenyl, (C₂-C₆)-Alkinyl, (C₁-C₆)-Alkoxy, HO-(C₁-C₆)- Alkyl, (C₁-C₆)-Alkoxy-(C₁-C₆)-alkyl, Phenyl, Benzyl, (C₁-C₄)-Alkylcarbonyl, SO-(C₁-C₆)-Alkyl, wobei in den Alkyl- und Alkoxyresten ein, mehrere, oder alle Wasserstoff(e) durch Fluor ersetzt sein können;
- A: (C₀-C₁₅)-Alkandiyl, wobei ein oder mehrere C-Atom(e) des Alkandiylrests unabhängig voneinander durch -O-, -(C=O)-, -CH=CH-, -C≡C-, -S-, - CH(OH)-, -CHF-, -CF₂-, -(S=O)-, -(SO₂)-, -N((C₁-C₆)-Alkyl)-, -N((C₁-C₆)- Alkyl-Phenyl)- oder -NH- ersetzt sein können;
- n: eine Zahl 2 oder 3;
- Cyc1: ein 5 bis 6 gliedriger gesättigter, teilweise gesättigter oder ungesättigter Ring, wobei 1 C-Atom durch O oder S ersetzt sein kann;
- R3, R4, R5: Wasserstoff, F, Cl, Br, J, OH, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂, (C₁- C₈)-Alkyl, (C₂-C₆)-Alkenyl, (C₂-C₆)-Alkinyl, (C₁-C₁₂)-Alkoxy, HO-(C₁-C₆)- Alkyl, (C₁-C₆)-Alkoxy-(C₁-C₆)-alkyl, (C₁-C₄)-Alkyl-Phenyl, (C₁-C₄)-Alkoxy- Phenyl, S-(C₁-C₆)-Alkyl, SO-(C₁-C₆)-Alkyl, wobei in den Alkyl- und Alkxoyresten ein, mehrere, oder alle Wasserstoff(e) durch Fluor ersetzt sein können; oder
- R3 und R4: gemeinsam mit den sie tragenden C-Atomen einen 5 bis 7 gliedrigen, gesättigten, teilweise oder vollständig ungesättigten Ring Cyc2, wobei 1 oder 2 C-Atom(e) des Ringes auch durch N, O oder S ersetzt sein können und Cyc2 gegebenenfalls durch (C₁-C₆)-Alkyl, (C₂-C₅)-Alkenyl, (C₂-C₅)- Alkinyl, wobei jeweils eine CH₂-Gruppe durch O ersetzt sein kann, oder durch F, Cl, OH, CF₃, NO₂, CN, COO(C₁-C₄)-Alkyl, CONH₂, CONH(C₁- C₄)-Alkyl, OCF₃ substituiert sein kann, und
- R5: Wasserstoff.

Besonders bevorzugt sind Verbindungen der Formel I, worin
- R1, R2: Wasserstoff, (C₁-C₆)-Alkyl, (C₁-C₄)-Alkoxy, HO-(C₁-C₄)-Alkyl, (C₁-C₄)- Alkoxy-(C₁-C₄)-alkyl, F, Cl, CF₃, OCF₃ , OCH₂CF₃ (C₁-C₄)-Alkyl-CF₂-,
- R1, R2: Wasserstoff, (C₁-C₆)-Alkyl, (C₁-C₄)-Alkoxy, HO-(C₁-C₄)-Alkyl, (C₁-C₄)- Alkoxy-(C₁-C₄)-alkyl, F, Cl, CF₃, OCF₃, OCH₂CF₃ (C₁-C₄)-Alkyl-CF₂-, Phenyl, Benzyl, (C₁-C₄)-Alkylcarbonyl, (C₂-C₄)-Alkenyl, (C₂-C₄)-Alkinyl, COO(C₁-C₄)-Alkyl;
- A: -CH=CH-CH₂- oder (C₁-C₄)-Alkandiyl, wobei eine oder zwei CH₂-Gruppen auch ersetzt sein können durch -(C=O)-, -CH=CH-, -CH(OH)-, -NH-, - CHF-, -CF₂-, -O-;
- n: eine Zahl 2 oder 3;
- Cyc1: ungesättigter Ring, wobei 1 C-Atom durch O oder S ersetzt sein kann;
- R3,R4,R5: Wasserstoff, F, Cl, Br, J, NO₂, OH, CN, (C₁-C₆)-Alkyl, (C₁-C₈)-Alkoxy, OCF₃, OCH₂CF₃ , S-(C₁-C₄)-Alkyl, COOH , HO-(C₁-C₄)-Alkyl, (C₁-C₄)- Alkoxy-(C₁-C₄)-alkyl, (C₁-C₂)-Alkyl-Phenyl, (C₁-C₂)-Alkoxy-Phenyl, oder
- R3 und R4: gemeinsam -CH=CH-O-, -CH=CH-S-, -O-(CH₂)ₚ-O-, mit p = 1 oder 2, -O- CF₂-O-, -CH=CH-CH=CH- und
- R5: Wasserstoff bedeuten.

Besonders bevorzugt ist ferner eine Zusammensetzung enthaltend eine Verbindung der Formel I, worin R2 Wasserstoff bedeutet.

Ganz besonders bevorzugt ist eine Zusammensetzung enthaltend eine Verbindung der Formel I, worin
- R1: Wasserstoff, CF₃, (C₁-C₄)-Alkyl, Phenyl,
- R2: Wasserstoff,
- A: -CH₂-, -C₂H₄-, -C₃H₆, -CH(OH)-, -(C=O)-, -CH=CH-, -CH=CH-CH₂-, - CO-CH₂-CH₂- oder -CO-NH-CH₂-;
- n: eine Zahl 2 oder 3;
- Cyc1: ungesättigter Ring, wobei 1 C-Atom durch S ersetzt sein kann;
- R3,R4,R5: Wasserstoff, F, Cl, J, NO₂. OH, CN, (C₁-C₆)-Alkyl, (C₁-C₈)-Alkoxy, O-CH₂- Phenyl, OCF₃, S-CH₃, COOH oder
- R3 und R4: gemeinsam -CH=CH-O-, -O-(CH₂)ₚ-O-, mit p = 1 oder 2, -O-CF₂-O-, - CH=CH-CH=CH-, und
- R5: Wasserstoff bedeuten.

Besonders bevorzugt ist eine Zusammensetzung enthaltend eine Verbindung der Formel I, worin
- A: -CH₂- oder -CH₂-CH₂- bedeutet oder
- Cyc1: Phenyl bedeutet oder
- Cyc1: Thienyl bedeutet.

Ferner seien insbesondere eine Zusammensetzung enthaltend eine Verbindung der Formel I genannt, worin
- Cyc1: monosubstituiert ist, oder
- Cyc1: para-substituiert ist, oder
- Cyc1: meta-substituiert ist.

Die Erfindung bezieht sich auf eine Zusammensetzung enthaltend eine Verbindung Verbindungen der Formel I, in Form ihrer Racemate, racemischen Mischungen und reinen Enantiomere sowie auf ihre Diastereomere und Mischungen davon.

Die Alkylreste, einschließlich Alkoxy, Alkenyl und Alkinyl, in den Substituenten R1, R2, R3, R4 und R5 können sowohl geradkettig wie verzweigt sein.

Die Zuckerreste in den Verbindungen der Formel I sehen sowohl für L- als auch für D-Zucker in ihrer alpha(α)- und beta(β)-Form, wie z.B. Allose, Altrose, Glucose, Mannose, Gulose, Idose, Galactose, Talose. Bevorzugt seien genannt: β-Glucose, β-Galactose, β-Allose und α-Mannose, besonders bevorzugt β-Glucose, β-Allose und α-Mannose, ganz besonders bevorzugt β-Glucose.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Des weiteren offenbart sind Verfahren zur Herstellung der Verbindungen der allgemeinen Formel I, die entsprechend den folgenden Reaktionsschemata A, B, C, D und E erhalten werden können:

### Verfahren A:

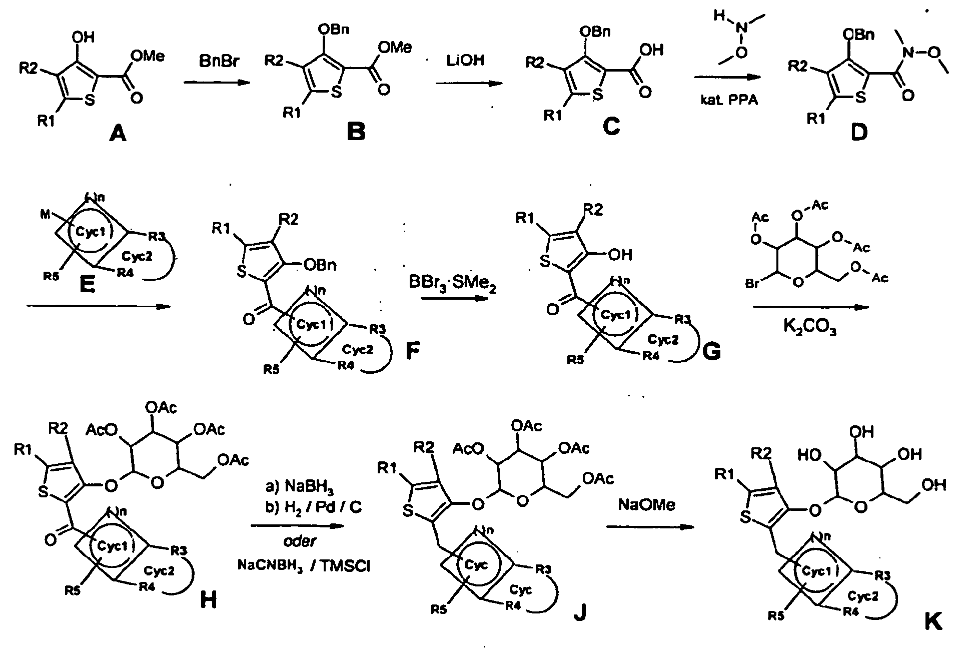

Es wird die Verbindung der allgemeinen Formel A, wobei R1 und R2 die oben beschriebenen Bedeutungen haben, in DMF mit CsCO₃ oder einer anderen geeigneten Base deprotoniert und anschließend mit Benzylbromid umgesetzt, wobei eine Verbindung der allgemeinen Formel B erhalten wird.

Die Verbindung B wird in einer Mischung aus Methanol, Tetrahydrofuran und Wasser gelöst und durch Umsetzung mit Lithiumhydroxid in die Verbindung der allgemeinen Formel C überführt.

Die Verbindung C wird mit N,O-Dimethylhydroxylamin unter Verwendung von Propanphosphonsäureanhydrid oder einem anderen geeigneten Aktivierungsreagenz zur Knüpfung von Amidbindungen in die Verbindung der allgemeinen Formel D überführt.

Die Verbindung D wird mit einer metallorganischen Verbindung der allgemeinen Formel E, wobei M = Li, MgCl, MgBr bedeutet, und Cyc1, Cyc2, n, R3, R4 , R5 die oben beschriebenen Bedeutungen haben, in Tetrahydrofuran gelöst und unter Eiskühlung mit einer Lewissäure (LA), vorzugsweise Zinntetrachlorid oder Aluminiumtrichlorid, versetzt und zur Verbindung der allgemeinen Formel F umgesetzt.

Zur Abspaltung des Benzylethers wird die Verbindung F entweder in Methylenchlorid gelöst und mit BBr₃-Dimethylsulfidkomplex umgesetzt, oder die Verbindung F wird in Methanol gelöst und unter einer Wasserstoffatmosphäre mit Palladium auf Kohle gerührt und man erhält die Verbindung der allgemeinen Formel G.

Die Verbindung G wird mit Essigsäure-4,5-diacetoxy-6-acetoxymethyl-2-bromo-tetrahydro-pyran-3-yl-ester und Kaliumcarbonat in einer Mischung aus Methylenchlorid und Wasser in die Verbindung der allgemeinen Formel H umgesetzt.

Die Verbindung H wird entweder zunächst mit Natriumborhydrid in einer Mischung aus Methanol und Tetrahydrofuran umgesetzt und anschließend in Ethanol unter Wasserstoffatmosphäre in Gegenwart von Palladium auf Kohle in die Verbindung der allgemeinen Formel J überführt, oder die Verbindung H wird in Acetonitril gelöst und in einer Mischung aus Natriumcyanoborhydrid und Chlortrimethylsilan direkt zur Verbindung der allgemeinen Formel J umgesetzt.

Die Verbindung J wird in Methanol gelöst und mit Natriummethanolat umgesetzt, wobei die Verbindung der allgemeinen Formel K erhalten wird.

Mit diesem Verfahren werden die Verbindungen der Beispiele 51 bis 54 synthetisiert.

### Verfahren B:

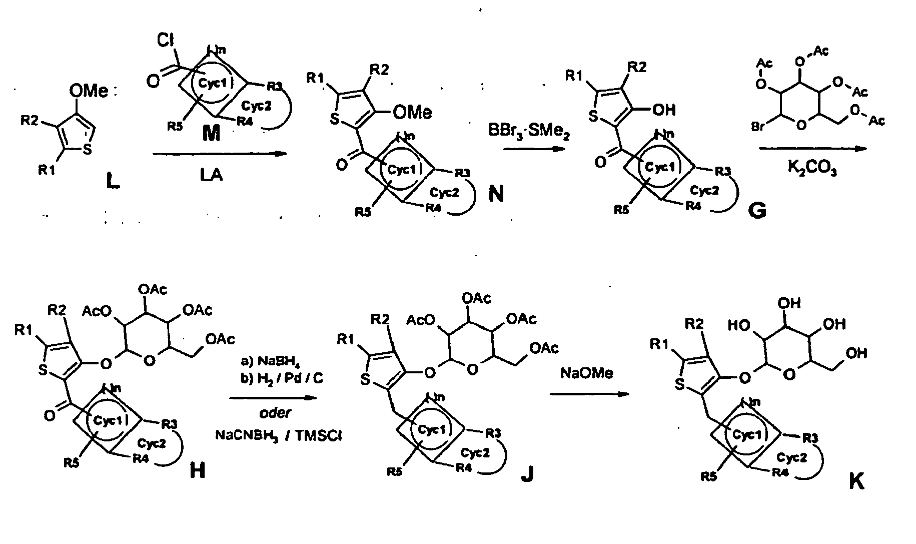

Die Verbindung der allgemeinen Formel L, wobei R1 und R2 die oben beschriebenen Bedeutungen haben, wird in Methylenchlorid gelöst und unter Eiskühlung mit einer Verbindung der allgemeinen Formel M, wobei Cyc1, Cyc2, n, R3, R4 , R5 die oben beschriebenen Bedeutungen haben, zur Verbindung der allgemeinen Formel N umgesetzt.

Die Verbindung N wird in Methylenchlorid gelöst und mit BBr₃-Dimethylsulfidkomplex umgesetzt, und man erhält so die Verbindung der allgemeinen Formel G.

Die Verbindung G wird mit Essigsäure-4,5-diacetoxy-6-acetoxymethyl-2-bromo-tetrahydro-pyran-3-yl-ester und Kaliumcarbonat in einer Mischung aus Methylenchlorid und Wasser in die Verbindung der allgemeinen Formel H umgesetzt.

Die Verbindung H wird entweder zunächst mit Natriumborhydrid in einer Mischung aus Methanol und Tetrahydrofuran umgesetzt und anschließend in Ethanol unter Wasserstoffatmosphäre in Gegenwart von Palladium auf Kohle in die Verbindung der allgemeinen Formel J überführt, oder die Verbindung H wird in Acetonitril gelöst und in einer Mischung aus Natriumcyanoborhydrid und Chlortrimethylsilan direkt zur Verbindung der allgemeinen Formel J umgesetzt.

Die Verbindung J wird in Methanol gelöst und mit Natriummethanolat umgesetzt, wobei die Verbindung der allgemeinen Formel K erhalten wird.

Mit diesem Verfahren werden die Verbindungen der Beispiele 7 bis 34 synthetisiert.

### Verfahren C:

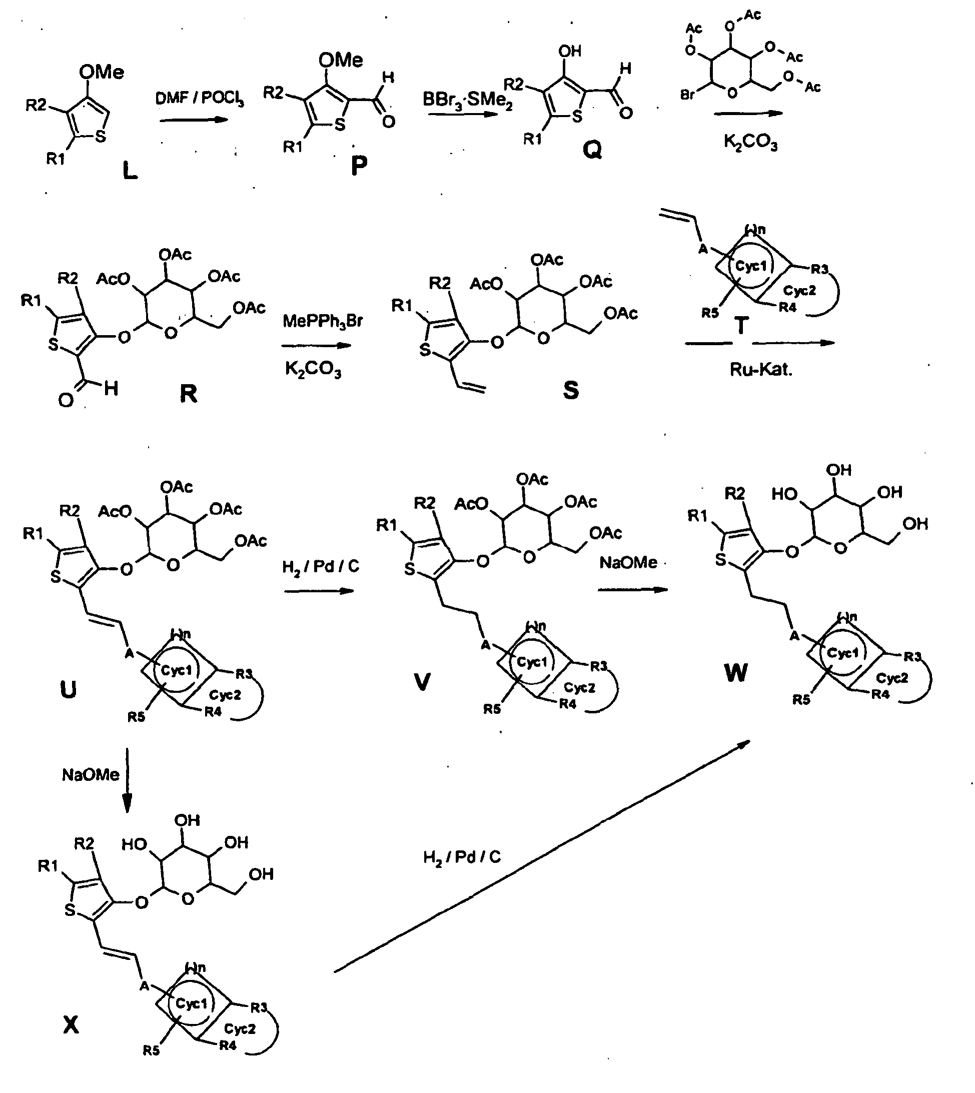

Es wird die Verbindung der allgemeinen Formel L, wobei R1 und R2 die oben beschriebenen Bedeutungen haben, in DMF gelöst und mit Phosphorylchlorid versetzt, wobei eine Verbindung der allgemeinen Formel P erhalten wird.

Die Verbindung P wird in Methylenchlorid gelöst und mit BBr₃-Dimethylsulfidkomplex umgesetzt, und man erhält so die Verbindung der allgemeinen Formel Q.

Die Verbindung Q wird mit Essigsäure-4,5-diacetoxy-6-acetoxymethyl-2-bromo-tetrahydro-pyran-3-yl-ester und Kaliumcarbonat in einer Mischung aus Methylenchlorid und Wasser zur Verbindung der allgemeinen Formel R umgesetzt.

Die Verbindung R wird in Dioxan gelöst und mit Methyltriphenylphosphoniumbromid und Kaliumcarbonat zur Verbindung der allgemeinen Formel S umgesetzt.

Die Verbindung S wird in Gegenwart des Ruthenium-Katalysators Tricyclohexylphosphin-[1,3-bis(2,4,6-trimethylphenyl)-4,5-dihydroimidazol-2-ylidene][benzyliden]ruthenium(IV)dichlorid in Dichlormethan mit der Verbindung der allgemeinen Formel T, wobei A, Cyc1, Cyc2, n, R3, R4 , R5 die oben beschriebenen Bedeutungen haben, zur Verbindung der allgemeinen Formel U umgesetzt.

Die Verbindung U wird in Methanol gelöst und mit Natriummethanolat umgesetzt, wobei die Verbindung der allgemeinen Formel X erhalten wird.

Alternativ kann die Verbindung U in Methanol unter Wasserstoffatmosphäre in Gegenwart von Palladium auf Kohle in die Verbindung der allgemeinen Formel V überführt werden.

Die Verbindung V wird in Methanol gelöst und mit Natriummethanolat umgesetzt, wobei die Verbindung der allgemeinen Formel W erhalten wird.

Alternativ kann W auch durch Hydrogenolyse von X erhalten werden. Dazu wird X in Methanol und in Gegenwart von Palladium auf Kohle unter Wasserstoffatmosphäre behandelt.

Mit diesem Verfahren werden die Verbindungen der Beispiele 36 bis 50 synthetisiert.

### Verfahren D:

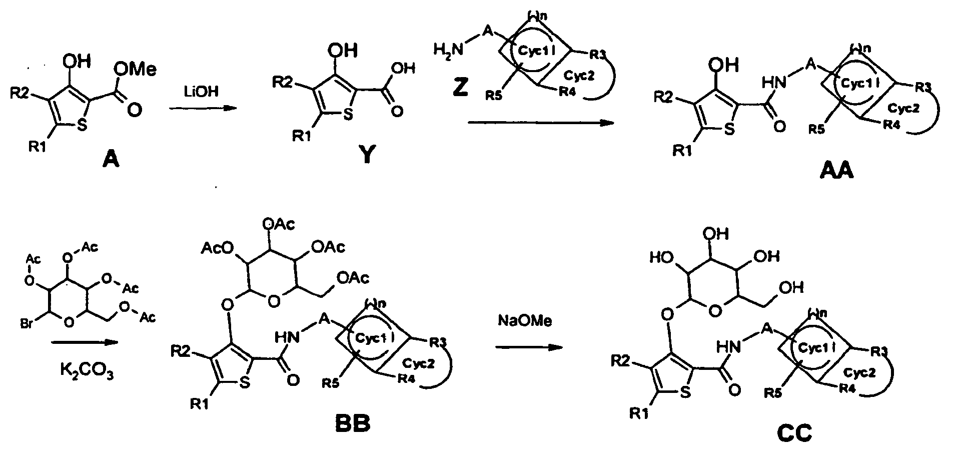

Es wird die Verbindung der allgemeinen Formel A, wobei R1 und R2 die oben beschriebenen Bedeutungen haben, in einer Mischung aus Methanol, Tetrahydrofuran und Wasser gelöst und durch Umsetzung mit Lithiumhydroxid in die Verbindung der allgemeinen Formel Y überführt.

Die Verbindung Y wird mit einer Verbindung der allgemeinen Formel Z, wobei A, Cyc1, Cyc2, n, R3, R4 , R5 die oben beschriebenen Bedeutungen haben, in Tetrahydrofuran gelöst und unter Eiskühlung der Verbindung unter Verwendung von Propanphosphonsäureanhydrid oder einem anderen geeigneten Aktivierungsreagenz zur Knüpfung von Amidbindungen in die Verbindung der allgemeinen Formel AA überführt.

Die Verbindung AA wird mit Essigsäure-4,5-diacetoxy-6-acetoxymethyl-2-bromo-tetrahydro-pyran-3-yl-ester und Kaliumcarbonat in einer Mischung aus Methylenchlorid und Wasser in die Verbindung der allgemeinen Formel BB umgesetzt.

Die Verbindung BB wird in Methanol gelöst und mit Natriummethanolat umgesetzt, wobei die Verbindung der allgemeinen Formel K erhalten wird.

Mit diesem Verfahren werden die Verbindungen der Beispiele 55 bis 58 synthetisiert.

### Verfahren E:

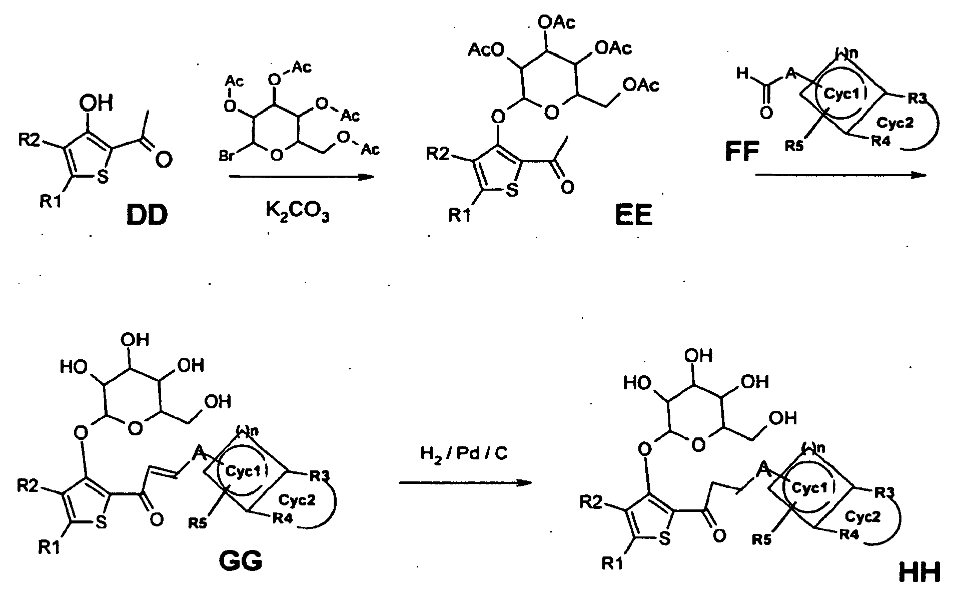

Die Verbindung DD wird mit Essigsäure-4,5-diacetoxy-6-acetoxymethyl-2-bromo-tetrahydro-pyran-3-yl-ester und Kaliumcarbonat in einer Mischung aus Methylenchlorid und Wasser in die Verbindung der allgemeinen Formel EE umgesetzt.

Die Verbindung EE wird in Methanol gelöst und mit Natriummethanolat in Methanol versetzt. Eine Verbindung der allgemeinen Formel FF, wobei A, Cyc1, Cyc2, n, R3, R4, R5 die oben beschriebenen Bedeutungen haben, wird zugegeben und man erhält eine Verbindung der allgemeinen Formel GG.

Die Verbindung GG wird in Methanol unter Wasserstoffatmosphäre in Gegenwart von Palladium auf Kohle in die Verbindung der allgemeinen Formel HH überführt.

Mit diesem Verfahren werden die Verbindungen der Beispiele 1 bis 6 synthetisiert.

Andere Verbindungen der Formel I können entsprechend oder nach bekannten Verfahren hergestellt werden.

Die Verbindung(en) der Formel (I) können auch in Kombination mit weiteren Wirkstoffen verabreicht werden.

Als weitere Wirkstoffe für die Kombinationspräparate sind geeignet:
Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.
Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMG-CoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in WO 00/64888, WO 00/64876, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. Cl-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylharnstoff, wie z.B. Tolbutamid, Glibenclamid, Glipizid oder Glimepirid verabreicht.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.
Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) , Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00 / 63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.
Bei einer Ausführungsform ist der weitere Wirkstoff Fenfluramin oder Dexfenfluramin.
Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

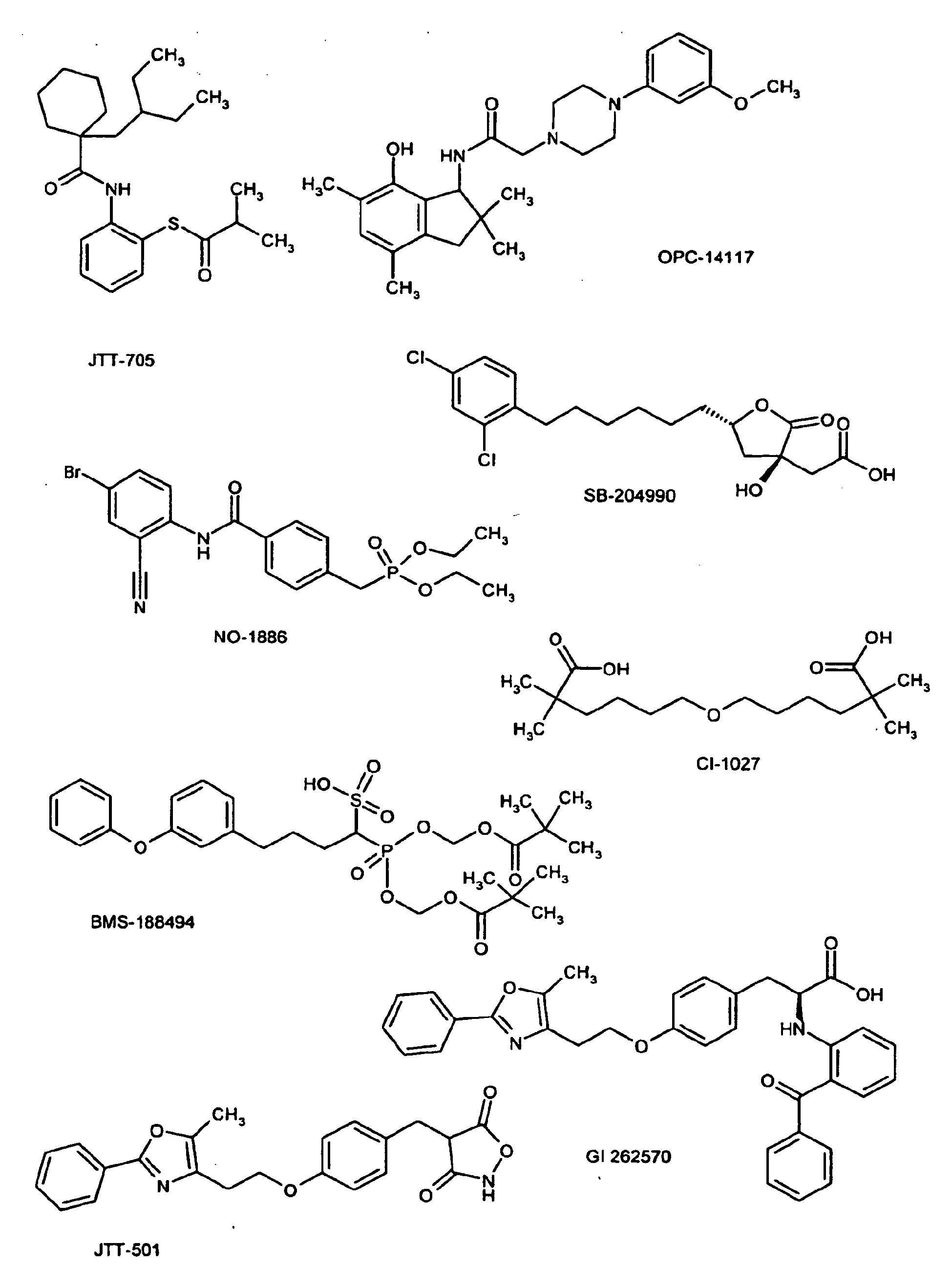

Die nachfolgend aufgeführten Beispiele dienen zur Erläuterung der Erfindung, ohne diese jedoch einzuschränken

**Tabelle 1: Verbindungen der Formel I**

| | | | | | |
|---|---|---|---|---|---|
| 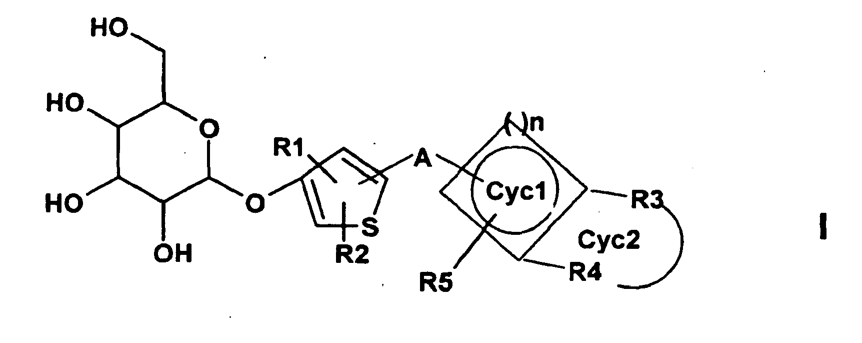 | | | | | |

| **Bsp.** | **R1, R2** | **A** (Verknüpfung in 2-Position Thienyl) | **Cyc1** | **R3, R4, R5** | **MS*** |
|---|---|---|---|---|---|
| 1 | H, H | -CO-CH₂-CH₂- | Ph | 4-O-CH₃, H, H | ok |
| 2 | H, H | -CO-CH₂-CH₂- | Ph | 3-O-(CH₂)₂-O-4, H | ok |
| 3 | H, H | -CO-CH₂-CH₂- | Ph | 3-O-CH₂-O-4, H | ok |
| 4 | H, H | -CO-CH₂-CH₂- | Ph | 3-CH=CH-O-4, H | ok |
| 5 | H, H | -CO-CH₂-CH₂- | 3-Thiophen | H, H, H | ok |
| 6 | H, H | -CO-CH₂-CH₂- | 2-Thiophen | H, H, H | ok |
| 7 | H, H | -CH₂- | Ph | 4-O-CH₃, H, H | ok |
| 8 | H, H | -CO- | Ph | 4-O-CH₃, H, H | ok |
| 9 | H, H | -CH₂- | Ph | H, H, H | ok |
| 10 | H, H | -CH(OH)- | Ph | H, H, H | ok |
| 11 | H, H | -CH₂- | Ph | 4-O-C₂H₅, H, H | ok |
| 12 | H, H | -CH₂- | Ph | 3-O-CH₃, 4-O-CH₃, H | ok |
| 13 | H, H | -CH₂- | Ph | 4-O-C₇H₁₀, H, H | ok |
| 14 | H, H | -CH₂- | Ph | 4-F, H, H | ok |
| 15 | H, H | -CH₂- | Ph | 4-I, H, H | ok |
| 16 | H, H | -CH₂- | Ph | 4-NO₂, H, H | ok |
| 17 | H, H | -CH₂- | Ph | 4-CH₃, H, H | ok |
| 18 | H, H | -CH₂- | Ph | 3-CH₃, H, H | ok |
| 19 | H, H | -CH₂- | Ph | 2-CH₃, H, H | ok |
| 20 | H, H | -CH₂- | Ph | 4-C₂H₅, H, H | ok |
| 21 | H, H | -CH₂- | Ph | 3-CH₃, 4-O-CH₃, 5-CH₃ | ok |
| 22 | H, H | -CH₂- | Ph | 3-O-CF₂-O-4, H | ok |
| 23 | H, H | -CH₂- | Ph | 4-C₃H₇, H, H | ok |
| 24 | H, H | -CH₂- | Ph | 4-C(CH₃)₃, H, H | ok |
| 25 | H, H | -CH₂- | Ph | 4-OH, H, H | ok |
| 26 | H, H | -CH₂- | Ph | 4-O-CH₂-Ph, H, H | ok |
| 27 | H, H | -CH₂- | 3-Thiophen | H, H, H | ok |
| 28 | H, H | -CH₂- | 2-Thiophen | 4-CH=CH-CH=CH-5, H | ok |
| 29 | H, H | -CH₂- | Ph | 3-O-CH₃, H, H | ok |
| 30 | H, H | -CH₂- | Ph | 4-CN, H, H | ok |
| 31 | H, H | -CH₂- | Ph | 3-O-CH₂-O-4, H, H | ok |
| 32 | H, H | -CH₂- | Ph | 4-S-CH₃, H, H | ok |
| 33 | H, H | -CH₂- | Ph | 4-O-C₄H₉, H, H | ok |
| 34 | H, H | -CH₂- | Ph | 4-OCF₃, H, H | ok |
| 35 | H, H | -CH₂- | Ph | 4-COOH, H, H | ok |
| 36 | H, H | -CH₂-CH₂- | Ph | 4-O-CH₃, H, H | ok |
| 37 | H, H | -CH=CH- | Ph | 4-O-CH₃, H, H | ok |
| 38 | H, H | -CH=CH- | Ph | 4-F, H, H | ok |
| 39 | H, H | -CH=CH- | Ph | 4-Cl, H, H | ok |
| 40 | H, H | -CH=CH- | Ph | 4-O-C₂H₅, H, H | ok |
| 41 | H, H | -CH=CH- | Ph | 4-CH₃, H, H | ok |
| 42 | H, H | -CH=CH- | Ph | 4-OH, H, H | ok |
| 43 | H, H | -CH₂-CH₂- | Ph | 4-F, H, H | ok |
| 44 | H, H | -CH₂-CH₂- | Ph | 4-Cl, H, H | ok |
| 45 | H, H | -CH₂-CH₂- | Ph | 4-O-C₂H₅, H, H | ok |
| 46 | H, H | -CH₂-CH₂- | Ph | 4-CH₃, H, H | ok |
| 47 | H, H | -CH₂-CH₂- | Ph | 4-OH, H, H | ok |
| 48 | H, H | -CH=CH-CH₂- | Ph | 4-O-CH₃, H, H | ok |
| 49 | H, H | -CH₂-CH₂-CH₂- | Ph | 4-O-CH₃, H, H | ok |
| 50 | H, H | -CH=CH-CH₂- | Ph | 3-O-CH₂-O-4, H | ok |
| 51 | 5-CH(CH₃)₂, H | -CH₂- | Ph | 4-O-CH₃, H, H | ok |
| 52 | 5-Ph, H | -CH₂- | Ph | 4-O-CH₃, H, H | ok |
| 53 | 5-CH₃, H | -CH₂- | Ph | 4-O-CH₃, H, H | ok |
| 54 | 5-CF₃, H | -CH₂- | Ph | 4-O-CH₃, H, H | ok |
| 55 | H, H | -CO-NH-CH₂- | Ph | H, H, H | ok |
| 56 | H, H | -CO-NH-CH₂- | Ph | 4-O-CH₃, H, H | ok |
| 57 | H, H | -CO-NH-CH₂- | Ph | 3-O-CH₂-O-4, H | ok |
| 58 | H, H | -CO-NH-CH₂- | Ph | 4-O-CF₃, H, H | ok |

| | | | | | |
|---|---|---|---|---|---|
| * Unter der Angabe "MS ist ok" wird verstanden, dass ein Massenspektrum oder HPLC/MS gemessen wurde und in diesem der Molpeak M+1 (MH⁺) und/oder M+18 (MNH₄⁺) und/oder M+23 (MNa⁺) nachgewiesen wurde | | | | | |

Die Verbindungen der Formel I zeichnen sich durch günstige Wirkungen auf den Glucosestoffwechsel aus, sie senken insbesondere den Blutzuckerspiegel und sind zur Behandlung von Typ 1 und Typ 2 Diabetes geeignet. Die Verbindungen können daher allein oder in Kombination mit weiteren Blutzucker-senkenden Wirkstoffen (Antidiabetika) eingesetzt werden.
Die Verbindungen der Formel I eignen sich weiterhin zur Prävention und Behandlung von Diabetischen Spätschäden, wie z.B. Nephropathie, Retinopathie, Neuropathie sowie Syndrom X, Obesitas, Herzinfarkt, peripheren arteriellen Verschlusskrankheiten, Thrombosen, Arteriosklerose, Entzündungen, Immunkrankheiten, Autoimmunkrankheiten, wie z.B. AIDS, Asthma, Osteoporose, Krebs, Psoriasis, Alzheimer, Schizophrenie und Infektionskrankheiten, bevorzugt ist die Behandlung von Typ 1 und Typ 2 Diabetes sowie die Prävention und Behandlung von Diabetischen Spätschäden, Syndrom X und Obesitas.

Die Wirksamkeit der Verbindungen wurde wie folgt getestet:

### Präparation von Bürstensaummembran-Vesikeln aus dem Dünndarm von Kaninchen, Ratte und Schwein

Die Präparation von Bürstensaummembran-Vesikeln aus den Darmzellen des Dünndarms erfolgte mit der sog. Mg²⁺-Präzipitationsmethode. Die Mucosa aus dem Dünndarm wurde abgeschabt und in 60 ml eiskaltem Tris/HCl-Puffer (ph 7,1) / 300 mM Mannit, 5 mM EGTA suspendiert. Nach dem Verdünnen auf 300 ml mit eiskaltem destilliertem Wasser wurde mit einem Ultraturrax (18-Stab, IKA Werk Staufen, BRD) 2 x 1 Minute bei 75 % max. Leistung unter Eiskühlung homogenisiert. Nach Zugabe von 3 ml 1 M MgCl₂-Lösung (Endkonzentration 10 mM) lässt man exakt 15 Minuten bei 0° C stehen. Durch die Zugabe von Mg²⁺ aggregieren die Zell-membranen und präzipitieren, mit Ausnahme der Bürstensaummembranen. Nach einer 15-minütigen Zentrifugation bei 3 000 x g (5 000 rpm, SS-34-Rotor) wird der Niederschlag verworfen und der Überstand, der die Bürstensaummembranen enthält, 30 Minuten bei 26 700 x g (15 000 rpm, SS-34-Rotor) zentrifugiert. Der Überstand wird verworfen, der Niederschlag in 60 ml 12 mM Tris/HCl-Puffer (pH 7,1) / 60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M MgCl₂-Lösung und 15-minütiger Inkubation bei 0°C wird erneut 15 Minuten bei 3 000 x g zentrifugiert. Der Überstand wird anschließend nochmals 30 Minuten bei 46 000 x g (20 000 rpm, SS-34-Rotor) zentrifugiert. Der Niederschlag wird in 30 ml 20 mM Tris/Hepes-Puffer (pH 7,4) / 280 mM Mannit aufgenommen und durch 20 Hübe in einem Potter Elvejhem Homogenisator bei 1 000 rpm homogen resuspendiert. Nach 30-minütiger Zentrifugation bei 48 000 x g (20 000 rpm, SS-34-Rotor) wurde der Niederschlag in 0,5 bis 2 ml Tris/Hepes-Puffer (pH 7,4) / 280 mM Mannit (Endkonzentration 20 mg/ml) aufgenommen und mit Hilfe einer Tuberkulinspritze mit einer 27 Gauge-Nadel resuspendiert.
Die Vesikel wurden entweder unmittelbar nach der Präparation für Markierungs- oder Transportuntersuchungen verwendet oder wurden bei - 196°C in 4 mg Portionen in flüssigem Stickstoff aufbewahrt.
Für die Präparation von Bürstensaummembranvesikeln aus Rattendünndarm wurden 6 bis 10 männliche Wistar-Ratten (Tierzucht Kastengrund, Aventis Pharma), durch zervikale Dislokation getötet, die Dünndärme entnommen und mit kalter isotonischer Kochsalzlösung gespült. Die Därme wurden aufgeschnitten und die Mucosa abgeschabt. Die Aufarbeitung zur Isolierung von Bürstensaummembranen erfolgte wie oben beschrieben. Zur Abtrennung von Cytoskelettanteilen wurden die Bürstensaummembranvesikel aus Rattendünndarm mit KSCN als chaotropem Ion behandelt.

Für die Präparation von Bürstensaummembranen aus Kaninchendünndarm wurden Kaninchen durch intravenöse Injektion von 0,5 ml einer wässrigen Lösung von 2,5 mg Tetracain-HCl, 100 mg m-Butramid und 25 mg Mebezoniumjodid getötet. Die Dünndärme wurden entnommen, mit eiskalter physiologischer Kochsalzlösung gespült und in Kunststoffbeutel unter Stickstoff bei - 80 °C eingefroren und 4 bis 12 Wochen gelagert. Zur Präparation der Membranvesikel wurden die eingefrorenen Därme bei 30°C im Wasserbad aufgetaut und anschließend die Mucosa abgeschabt. Die Aufarbeitung zu Membranvesikeln erfolgte wie oben beschrieben.

Zur Präparation von Bürstennsaummembranvesikeln aus Schweinedarm wurden Jejunumsegmente eines frisch geschlachteten Schweines mit eiskalter isotonischer Kochsalzlösung gespült und in Plastikbeuteln unter Stickstoff bei - 80°C eingefroren. Die Präparation der Membranvesikel erfolgte wie oben beschrieben.

### Präparation von Bürstensaummembranvesikeln aus dem Nierenkortex der Rattenniere

Die Präparation der Bürstensaummembranvesikel aus dem Kortex der Rattenniere erfolgte nach der Methode von Biber et al. Die Nieren von 6 bis 8 Ratten (200 bis 250 g) wurden entnommen und von jeder Niere wurde der Kortex als ca. 1 mm starke Schicht abgetragen. Die Nieren wurden in 30 ml eiskaltem 12 mM Tris/HCl-Puffer (pH 7,4) / 300mM Mannit aufgenommen und unter Eiskühlung 4 x 30 Sekunden mit einem Ultraturraxstab (Stufe 180 V) homogenisiert. Nach der Zugabe von 42 ml eiskaltem destilliertem Wasser wurden 850 µl einer 1 M MgCl₂-Lösung zugegeben. Nach 15-minütiger Inkubation bei 0°C wurde 15 Minuten bei 4 500 rpm (Sorvall SS-34-Rotor) zentrifugiert. Der Niederschlag wurde verworfen, der Überstand 30 Minuten bei 16 000 rpm zentrifugiert. Nach Resuspendierung des Niederschlags in 60 ml 6 mM Tris/HCl-Puffer (pH 7,4) / 150 mM Mannit / 2,5 mM EGTA durch 10 Hübe in einem Potter-Elvejhem Homogenisator (900 rpm) wurde nach Zugabe von 720 µl 1 mM MgCl₂-Lösung 15 Minuten bei 0°C inkubiert. Nach 15-minütiger Zentrifugation bei 4 500 rpm (SS-34-Rotor) wurde der resultierende Überstand 30 Minuten bei 16000 rpm zentrifugiert. Der Überstand wurde durch 10 Hübe in 60 ml 20 mM Tris/Hepes-Puffer (pH 7,4) / 280 mM Mannit homogenisiert und die entstehende Suspension anschließend 30 Minuten bei 20 000 rpm zentrifugiert. Der Niederschlag wurde mit Hilfe einer Tuberkulinspritze mit einer 27 Gauge-Nadel in 20 mM Tris/HCl-Puffer (pH 7,4) / 280 mM Mannit resuspendiert und auf eine Proteinkonzentration von 20 mg/ml eingestellt.

### Messung der Glukoseaufnahme durch Bürstensaummembranvesikel

Die Aufnahme von [¹⁴C]-markierter Glukose in Bürstensaummembranvesikel wurde mittels der Membranfiltrationsmethode gemessen. 10 µl der Bürstensaummembranvesikelsuspension in 10 mM Tris/Hepes-Puffer (pH 7.4)/300 mM Mannitol wurden bei 30°C zu 90 µl einer Lösung von 10 µM [¹⁴C]D-Glukose und den entsprechenden Konzentrationen der betreffenden Hemmstoffe (5-200 µM) in 10 mM Tris/Hepes-Puffer (pH 7.4)/ 100 mM NaCl/100 mM gegeben.
Nach 15 sec. Inkubation wurde der Transportprozess durch Zugabe von 1 ml eiskalter Stopplösung (10 mM Tris/Hepes-Puffer (pH 7.4)/ 150 mM KCI) angehalten und die Vesikelsuspension wurde sofort bei einem Vakuum von 25 bis 35 mbar über ein Membranfilter aus Cellulosenitrat (0,45 µm, 25 mm Durchmesser, Schleicher & Schüll) abgesaugt. Der Filter wurde mit 5 ml eiskalter Stopplösung nachgewaschen.
Jeder Messpunkt wurde als Doppel- oder Dreifachbestimmung ausgeführt.
Zur Messung der Aufnahme radioaktiv markierter Substrate wurde der Membranfilter in 4 ml eines entsprechenden Szintillators (Quickszint 361, Zinsser Analytik GmbH, Frankfurt am Main) aufgelöst und die Radioaktivität durch Flüssigkeisszintillationsmessung bestimmt. Die gemessenen Werte wurden nach Eichung des Gerätes mit Hilfe von Standardproben und nach Korrektur evtl. vorhandener Chemilumiszenz als dpm (Disintegrations per minute) erhalten.

Der Aktivitätsvergleich der Wirkstoffe wird anhand von IC₂₅ Daten durchgeführt, die im Transport-Assay an Nierenkortex- Bürstensaummembranvesikeln des Kaninchens für ausgewählte Substanzen erhalten wurden. (Die Absolutwerte können Spezies- und Versuchs-abhängig sein)

| Beispiel Nr. | IC25 [µM] |
|---|---|
| 5* | 13.9 |
| 6* | 9.9 |
| 7* | 1.1 |
| 9* | 1.4 |
| 11* | 1.3 |
| 13* | 3.5 |
| 34* | 1.0 |
| 43* | 2.2 |
| 44* | 0.9 |
| 45* | 2.9 |
| 47* | 1.6 |
| 50* | 4.7 |
| 54* | 1.4 |
| 56* | 2.8 |

| | |
|---|---|
| * β-D-Gluco-Form | |

### Nachfolgend wird die Herstellung verschiedener Beispiele detailliert beschrieben, die übrigen Verbindungen der Formel I wurden analog erhalten:

### Experimenteller Teil:

### Beispiel 1:

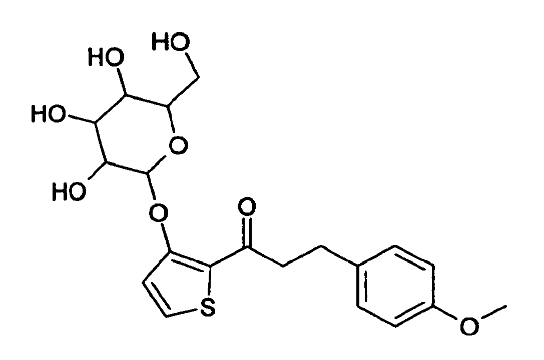

### 3-(4-Methoxy-phenyl)-1-[3-(3,4, 5-trihydroxy-6-hydroxymethyl-tetrahydro-pyran-2-yloxy)-thiophen-2-yl]-propan-1-on

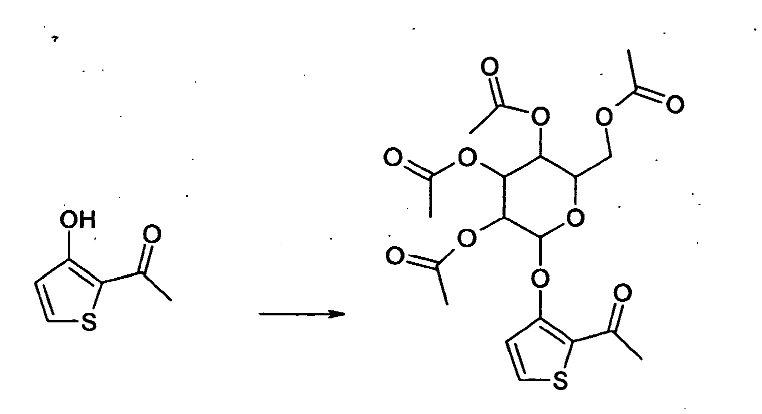

### a) Essigsäure-4,5-diacetoxy-6-acetoxymethyl-2-(2-acetyl-thiophen-3-yloxy)-tetrahydropyran-3-yl-ester

2 g 1-(3-Hydroxy-thiophen-2-yl)-ethanon werden in 120 ml Dichlormethan gelöst und mit 6.4 g Essigsäure-4,5-diacetoxy-6-acetoxymethyl-2-bromo-tetrahydro-pyran-3-yl-ester, 1.4 g Benzyl-tributylammoniumchlorid, 6.4 g Kaliumcarbonat und 1.2 ml Wasser 20 Std. bei 22°C gerührt. Man filtriert von unlöslichen Bestandteilen ab, engt ein und reinigt die Rohproduktmischung durch Säulenchromatographie (SiO₂, Ethylacetat / n-Heptan = 1:1). Man erhält das Produkt mit dem Molekulargewicht 472.5 (C₂₀H₂₄O₁₁S), MS (Cl): 473 (M+H⁺).

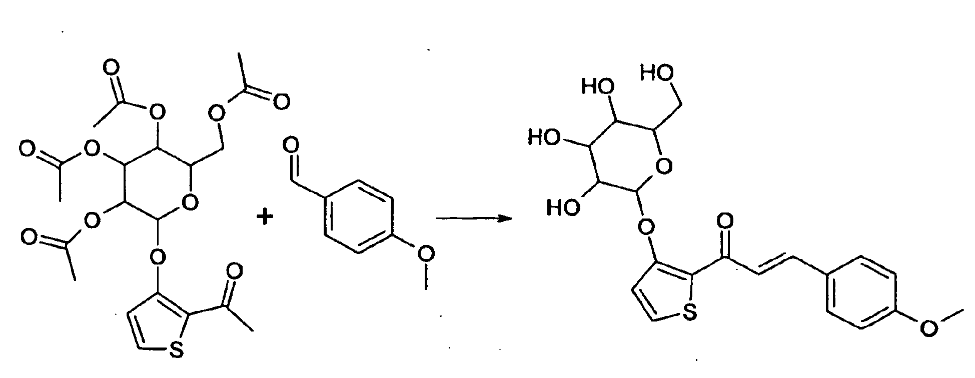

### b) 3-(4-Methoxy-phenyl)-1-[3-(3,4,5-trihydroxy-6-hydroxymethyl-tetrahydro-pyran-2-yloxy)-thiophen-2-yl]-propenon

472 mg Essigsäure-4,5-diacetoxy-6-acetoxymethyl-2-(2-acetyl-thiophen-3-yloxy)-tetrahydro-pyran-3-yl ester werden in 20 ml Methanol gelöst und mit 5 ml 1N NaOCH₃-Lösung in Methanol versetzt. Man gibt 410 mg 4-Methoxy-benzaldehyd dazu und rührt 20 Std. bei 22°C. Der Ansatz wird mit wenig verdünnter methanolischer Salzsäure neutralisiert, eingeengt und der Rückstand über eine Kieselgelsäule chromatographisch ( Dichlormethan/Methanol = 6:1) gereinigt. Man erhält das Produkt mit dem Molekulargewicht 422.5 (C₂₀H₂₂O₈S), MS (ESI): 423 (M+H⁺).

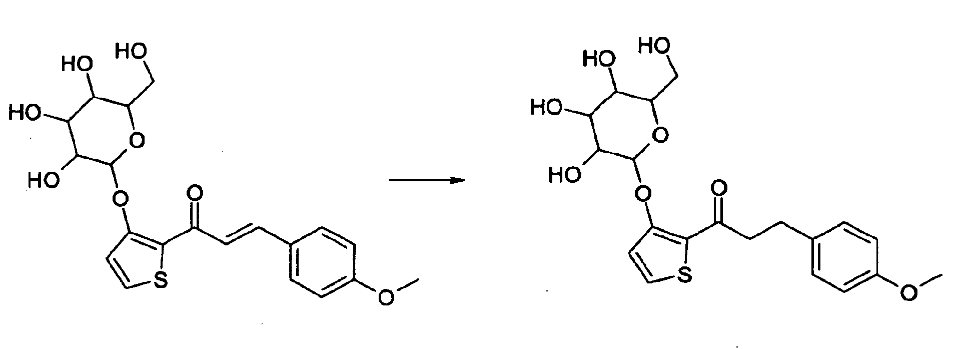

### c) 3-(4-Methoxy-phenyl)-1-[3-(3,4,5-trihydroxy-6-hydroxymethyl-tetrahydro-pyran-2-yloxy)-thiophen-2-yl]-propan-1-on

100 mg 3-(4-Methoxy-phenyl)-1-[3-(3,4,5-trihydroxy-6-hydroxymethyl-tetrahydro-pyran-2-yloxy)-thiophen-2-yl]-propenon werden gelöst in 10 ml Ethanol mit ca. 20 mg 5proz. Palladium auf Kohle in eine Schüttelente bei leichtem Überdruck hydriert (ca. 4 Std., DC-Kontrolle). Man filtriert vom Katalysator ab, engt ein und reinigt den Rückstand durch Säulenfiltration (SiO₂, Dichlormethan/Methanol = 6:1). Man erhält das Produkt mit dem Molekulargewicht 424.5 (C₂₀H₂₄O₈S), MS (ESI): 447 (M+Na⁺).

In der oben beschriebenen Synthesesequenz wurde α-D-Acetobromglucose als Essigsäure-4,5-diacetoxy-6-acetoxymethyl-2-bromo-tetrahydro-pyran-3-yl-ester verwendet. Man erhielt so das Glycosid des Beispiels 1 in der β-D-Gluco-Form. Dies gilt auch für alle nachfolgend beschriebenen Beispiele. Verwendet man hingegen α-D-Acetobromgalactose, so erhält man das Glycosid in β-D-Galacto-Form, verwendet man α-D-Acetobromallose, so erhält man das Glycosid in β-D-Allo-form oder verwendet man α-D-Acetobrommannose, so erhält man das Glycosid in α-D-Manno-Form.

Auf dem gleichen Syntheseweg wie vorstehend in Beispiel 1 beschrieben werden die nachfolgenden Beispiel-Substanzen 2 bis 6 hergestellt:

| 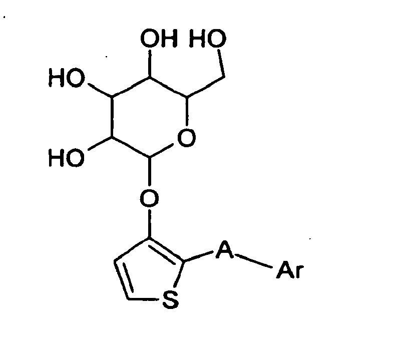 | | | |
|---|---|---|---|
| **Beispiel** | **A** | **Ar** | **MS oder LC/MS** |
| **2** | 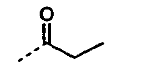 | 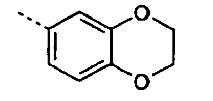 | **OK** |
| **3** | 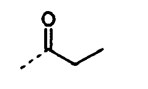 | 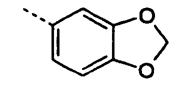 | **OK** |
| **4** | 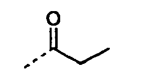 | 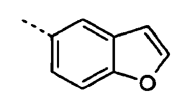 | **OK** |
| **5** | 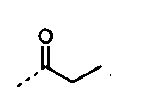 | 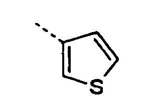 | **OK** |
| **6** | 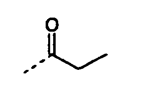 | 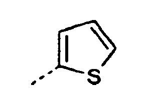 | **OK** |

### Beispiel 7:

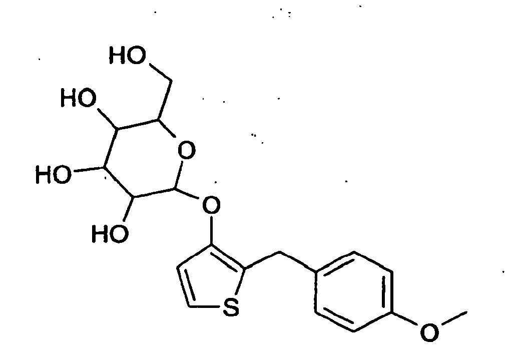

### 2-Hydroxymethyl-6-[2-(4-methoxy-benzyl)-thiophen-3-yloxy]-tetrahydro-pyran-3,4,5-triol

### Beispiel 8:

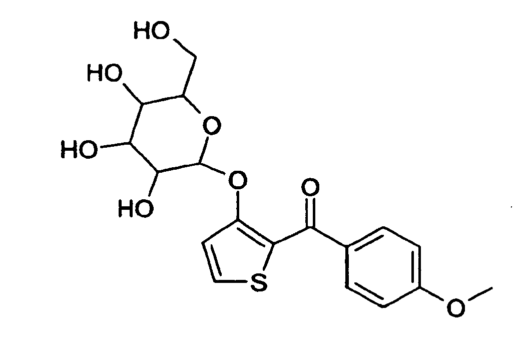

### (4-Methoxy-phenyl)-[3-(3,4,5-trihydroxy-6-hydroxymethyl-tetrahydro-pyran-2-yloxy)-thiophen-2-yl]-methanon

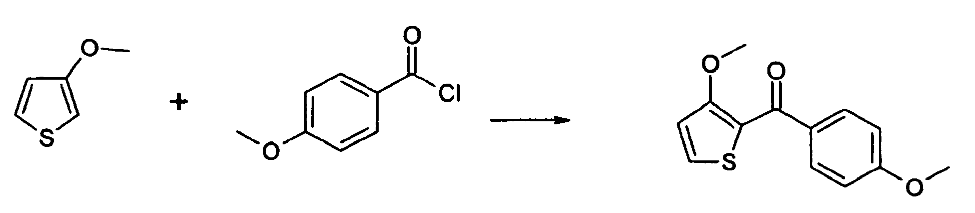

### a) (4-Methoxy-phenyl)-(3-methoxy-thiophen-2-yl)-methanon

Zu einer Lösung von 2.3 g 3-Methoxy-thiophen und 3.4 g 4-Methoxybenzoylchlorid in 50 ml Dichlormethan gibt man unter Eiskühlung 2.7 ml Zinntetrachlorid. Der Ansatz wird über Nacht bei Raumtemp. gerührt. Zur Aufarbeitung versetzt man mit 75 ml 2N Salzsäure und schüttelt dreimal mit Dichlormethan aus. Die vereinten organischen Phasen werden je zweimal mit 2N Natriumcarbonat-Lösung und Wasser gewaschen, dann entfernt man das Lösemittel i.Vak. und reinigt das Rohprodukt durch Säulenfiltration (SiO₂, Ethylacetat/n-Heptan = 1:2). Man erhält das Produkt mit dem Molekulargewicht 248.3 (C₁₃H₁₂O₃S), MS (Cl): 249 (M+H⁺)

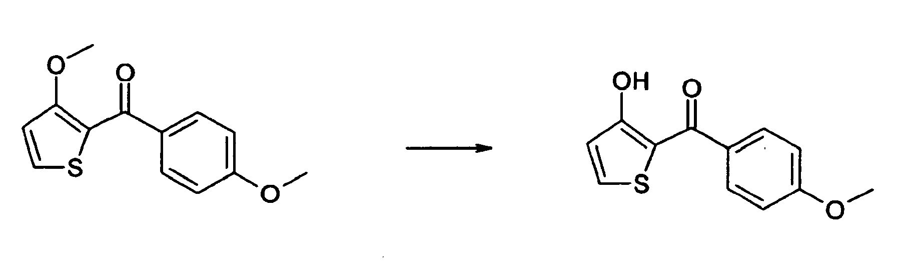

### b) (3-Hydroxy-thiophen-2-yl)-(4-methoxy-phenyl)-methanon

993 mg (4-Methoxy-phenyl)-(3-methoxy-thiophen-2-yl)-methanon werden in 20 ml trockenem Dichlormethan gelöst und mit 7 ml Bortribromid-Dimethylsulfid-Komplex versetzt. Die Mischung wird bis zur Vervollständigung der Umsetzung bei Raumtemp. gerührt (DC-Kontrolle). Danach gießt man auf Wasser und schüttelt mehrfach mit Dichlormethan aus. Die organische Phase wird getrocknet, eingeengt und der Rückstand wird durch Säulenchromatographie gereinigt (SiO₂, Ethylacetat/n-Heptan = 1:4). Man erhält das Produkt mit dem Molekulargewicht 234.3 (C₁₂H₁₀O₃S), MS (Cl): 235 (M+H⁺).

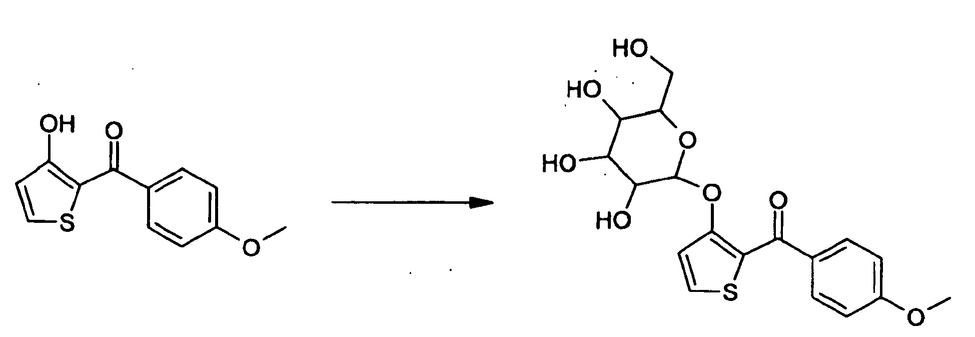

### c) (4-Methoxy-phenyl)-[3-(3,4,5-trihydroxy-6-hydroxymethyl-tetrahydro-pyran-2-yloxy)-thiophen-2-yl]-methanon = Beispiel 8

2.8 g (3-Hydroxy-thiophen-2-yl)-(4-methoxy-phenyl)-methanon werden in 350 ml Dichlormethan gelöst und mit 12.64g Essigsäure-3,4,5-triacetoxy-6-brom-tetrahydropyran-2-ylmethylester, 15.4 g Kaliumcarbonat, 3.6 g Benzyl-tributylammoniumchlorid und zuletzt 3 ml Wasser versetzt. Die Mischung wird 20h bei Raumtemp. intensiv gerührt. Nach beendeter Reaktion wird filtriert, eingeengt und der Rückstand über SiO₂ mit Ethylacetat/Heptan = 1:2 filtriert. Man entfernt das Lösemittel und nimmt den Rückstand in ca. 300 ml Methanol auf, versetzt mit 35 ml 1N NaOCH₃-Lösung in Methanol und rührt 1 Std. bei Raumtemp. Danach neutralisiert man mit 7proz. methanolischer Salzsäure (ca. 35 ml), setzt ca. 100 ml Laufmittelgemisch Dichlormethan/Methanol/konz.Ammoniak = 30:5:0.1 dazu und rührt 5 min. Danach wird eingeengt, der Rückstand mit gleichem Laufmittelgemisch aufgenommen und die Lösung vom unlöslichen Salz abgetrennt. Man erhält nach Chromatographie über Kieselgel das Produkt mit dem Molekulargewicht 396.42 (C₁₈H₂₀O₈S), MS (ESI): 397 (M+H⁺), 235 (M+H⁺- gluc).

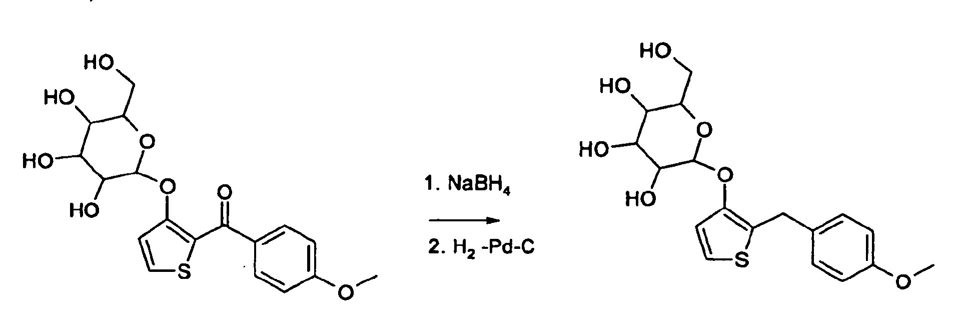

### d) 2-Hydroxymethyl-6-[2-(4-methoxy-benzyl)-thiophen-3-yloxy]-tetrahydro-pyran-3,4,5-triol = Beispiel 7

4.1 g (4-Methoxy-phenyl)-[3-(3,4,5-trihydroxy-6-hydroxymethyl-tetrahydro-pyran-2-yloxy)-thiophen-2-yl]-methanon werden in 200 ml Tetrahydrofuran und 20 ml Methanol gelöst und mit 500 mg Natriumborhydrid versetzt. Nach vollständiger Umsetzung (DC-Kontrolle, Dichlormethan/Methanol/konz. Ammoniak= 30:5:1; ca. 30 - 60 min) wird mit Wasser versetzt und dreimal mit Essigsäureethylester exrahiert. Die vereinten organischen Phasen werden über Magnesiumsulfat getrocknet und eingeengt. Man erhält 2-{2-[Hydroxy-(4-methoxy-phenyl)-methyl]-thiophen-3-yloxy}-6-hydroxymethyltetrahydro-pyran-3,4,5-triol als Rohprodukt, das durch Filtration über Kieselgel gereinigt werden kann.
Die gesamte Menge wird in ca. 800 ml trockenem Ethanol gelöst, und man sättigt die Lösung in einer Schüttelente mit Argon. Man gibt trockenes Palladium auf Kohle als Katalysator hinzu und hydriert die Mischung unter kräftigem Schütteln 6-7 Std. bei 22 °C und Normaldruck. Nach beendeter Umsetzung saugt man über eine Klärschicht ab und entfernt das Lösemittel i.Vak. Der Rückstand wird durch Säulenchromatographie (SiO₂, Dichlormethan/Methanol = 9:1) gereinigt. (Entwicklung der DC-Platten mit 10proz. Schwefelsäure). Man erhält das Produkt mit dem Molekulargewicht 382.44 (C₁₈H₂₂O₇S), MS (ESI): 383 (M+H⁺), 221 (M+H⁺-gluc).

Alternativ kann diese Verbindung auch auf folgendem Weg dargestellt werden:

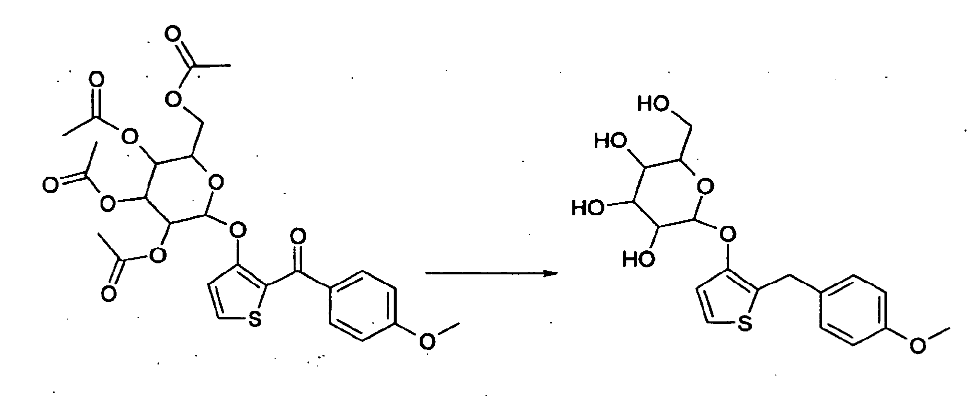

226 mg Essigsäure-3,4,5-triacetoxy-6-[2-(4-methoxy-benzyl)-thiophen-3-yloxy]-tetrahydro-pyran-2-yl-methylester werden in 4 ml Acetonitril gelöst und im Eisbad auf 0 °C gekühlt. 0.3 ml Trimethylchlorosilan und 151 mg Natriumcyanoborhydrid werden zugegeben, das Eisbad wird entfernt und die Reaktion wird 2 Std. gerührt. Die Reaktionsmischung wird mit 30 ml Dichlormethan verdünnt, über Celite filtriert und die organische Phase wird mit 20 ml gesättigter Natriumhydrogencarbonat- und 20 ml Natriumchloridlösung gewaschen. Der Rückstand wird durch Säulenchromatographie (SiO₂, Ethylacetat/n-Heptan = 1:2) gereinigt. Das Rohprodukt wird in Methanol aufgenommen und mit 1 ml Natriummethanolat-Lösung (10 mg / ml in Methanol) versetzt. Die Lösung wird 18 Std. bei 22 °C gerührt, mit Amberlyst 15 (H⁺-Form) versetzt, mit 10 ml Methanol verdünnt und filtriert. Der Rückstand wird mit 20 ml Methanol gewaschen, die organische Phase wird eingeengt und der Rückstand über Kieselgel chromatographiert. Man erhält 120 mg des Produkts mit dem Molekulargewicht 382.44 (C₁₈H₂₂O₇S), MS (ESI): 400 (M+NH₄⁺)

### Darstellung von (3-Methoxy-thiophen-2-yl)-(4-nitro-phenyl)-methanon:

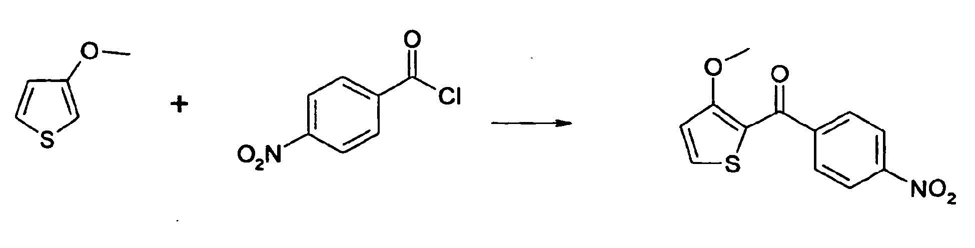

0,5 ml 3-Methoxythiophen werden in 50 ml Dichlormethan gelöst. 968 mg 4-Nitrobenzoylchlorid werden zugegeben und das Reaktionsgemisch wird im Eisbad auf 0°C gekühlt. Anschließend werden 696 mg Aluminiumtrichlorid zugegeben und die Reaktion wird 4 Std. bei 0°C gerührt. Das Reaktionsgemisch wird zu 100 ml Eiswasser gegeben, 15 Min. gerührt und mit 100 ml Dichlormethan versetzt. Die organische Phase wird abgetrennt, mit 50 ml 0,5-molarer Natriumhydroxidlösung und 50 ml gesättigte Natriumchloridlösung gewaschen, über Natriumsulfat getrocknet und eingeengt. Anschließend wird das erhaltene Gemisch säulenchromatographisch gereinigt (SiO₂, Essigester/n Heptan). Man erhält das Produkt mit dem Molekulargewicht 263.27 (C₁₂H₉NO₄S); MS (Cl): 264.25 (M+H⁺). (3-Methoxy-thiophen-2-yl)-(4-nitro-phenyl)-methanon wird anschließend, wie exemplarisch für Beispiel 7 beschrieben, in die Beispiel-Substanz 16 überführt.

Auf dem gleichen Syntheseweg werden folgende Beispiel-Substanzen 9 bis 34 hergestellt.

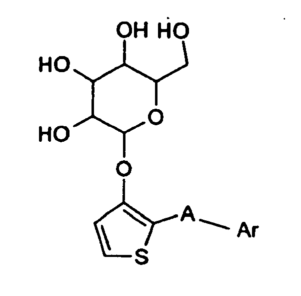

| **Beispiel** | **A** | **Ar** | **MS oder LC/MS** |
|---|---|---|---|
| **9** | CH₂ | 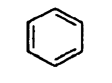 | **OK** |
| **10** | CHOH | 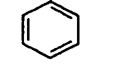 | **OK** |
| **11** | CH₂ | 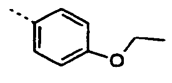 | **OK** |
| **12** | CH₂ | 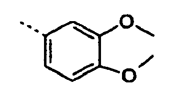 | **OK** |
| **13** | CH₂ | 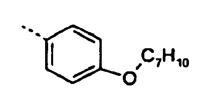 | **OK** |

| Beispiel | A | Ar | MS oder LC/MS |
|---|---|---|---|
| 14 | CH₂ | 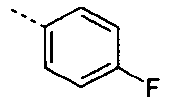 | OK |
| 15 | CH₂ | 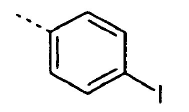 | OK |
| 16 | CH₂ | 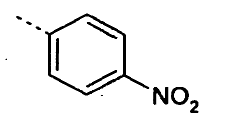 | OK |
| 17 | CH₂ | 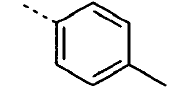 | OK |
| 16 | CH₂ | 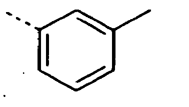 | OK |
| 19 | CH₂ | 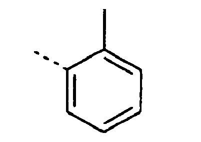 | OK |
| 20 | CH₂ | 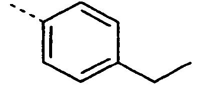 | OK |
| 21 | CH₂ | 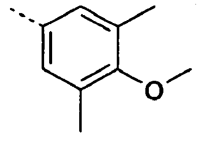 | OK |
| 22 | CH₂ | 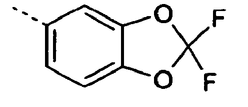 | OK |
| 23 | CH₂ | 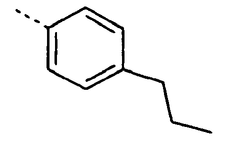 | OK |
| 24 | CH₂ | 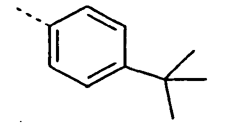 | OK |
| 25 | CH₂ | 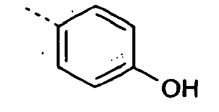 | OK |
| 26 | CH₂ | 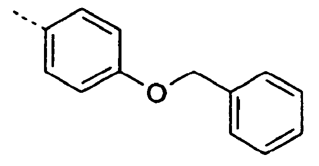 | OK |
| 27 | CH₂ | 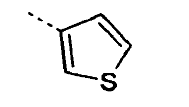 | OK |
| 28 | CH₂ | 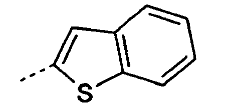 | OK |
| 29 | CH₂ | 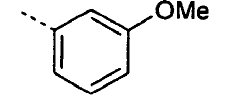 | OK |
| 30 | CH₂ | 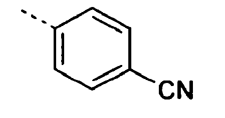 | OK |
| 31 | CH₂ | 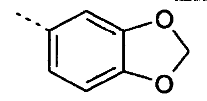 | OK |
| 32 | CH₂ | 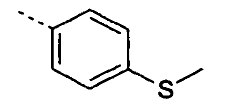 | OK |
| 33 | CH₂ | 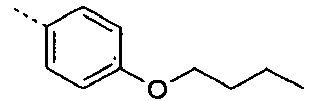 | OK |
| 34 | CH₂ | 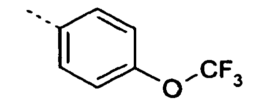 | OK |

Die Angabe MS/LCMS OK besagt, daß der Molpeak der angegebenen Verbindung als M+1 (MH⁺) und / oder als M+18 (MNH₄⁺) und / oder M + 23 (MNa⁺) erhalten wurde.

### Beispiel 35:

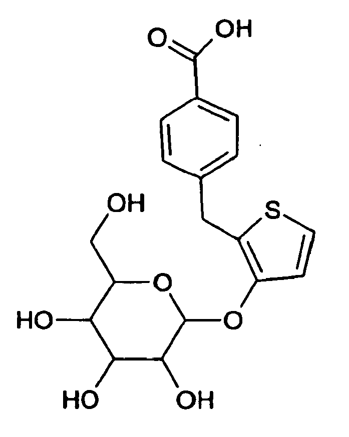

### 4-[3-(3,4,5-Trihydroxy-6-hydroxymethyl-tetrahydro-pyran-2-yloxy)-thiophen-2-ylmethyl]-benzoesäure

46 mg 4-[3-(3,4,5-Trihydroxy-6-hydroxymethyl-tetrahydro-pyran-2-yloxy)-thiophen-2-ylmethyl]-benzonitril werden in einer Mischung aus 5 ml Methanol und 2ml 25%iger Kaliumhydroxid-Lösung gelöst und 3 Std. auf 70° erwärmt. Die Lösung wird mit 10 ml Wasser verdünnt und mit 2N HCl neutralisiert. Die erhaltene Lösung wird gefriergetrocknet. Anschließend wird das Rohprodukt säulenchromatographisch gereinigt (SiO₂, Dichlormethan/Methanol/Essigsäure/Wasser = 8:2:0.1:0.1). Man erhält 45 mg des Produkts mit dem Molekulargewicht 396,42 (C₁₈H₂₀O₈S), MS (ESI): 414.45 (M+NH₄⁺).

### Beispiel 36:

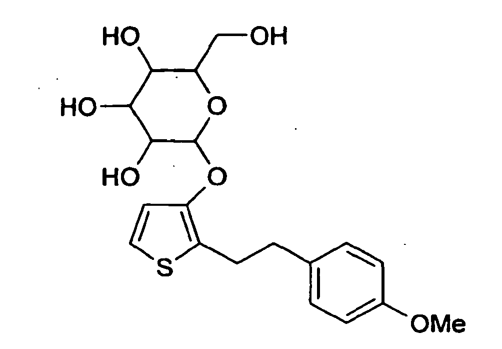

### 2-Hydroxymethyl-6-{2-[2-(4-methoxy-phenyl)-ethyl]-thiophen-3-yloxy}-tetrahydro-pyran-3,4,5-triol

### Beispiel 37:

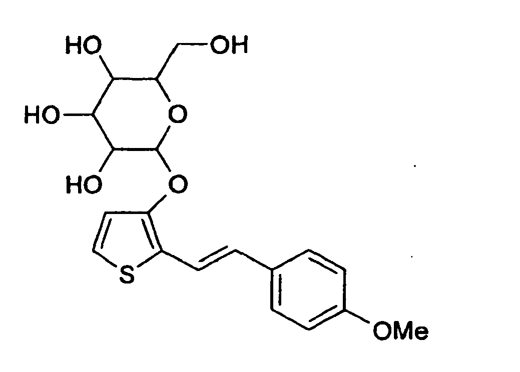

### 2-Hydroxymethyl-6-{2-[2-(4-methoxy-phenyl)-vinyl]-thiophen-3-yloxy}-tetrahydro-pyran-3,4,5-triol

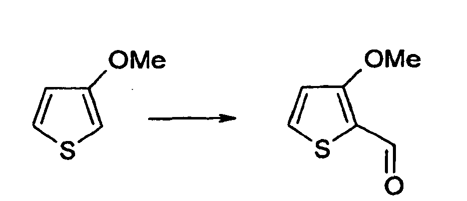

### a) 3-Methoxy-thiophen-2-carbaldehyd

1,03 ml 3-Methoxythiophen werden in 2.3 ml Dimethylformamid gelöst. Unter Eiskühlung werden 1,06 ml Phosphorylchlorid zugegeben. Nach 1 Std. wird die Reaktionslösung auf Eis gegeben, und die Lösung wird mit 5-molare NatriumhydroxidLösung neutralisiert. Die wässrige Phase wird 3mal mit je 25 ml Diethylether extrahiert, die vereinten organischen Phasen werden anschließend mit 50 ml gesättigter Natriumchlorid-Lösung gewaschen, über Natriumsulfat getrocknet und eingeengt. Man erhält 840 mg des Produkts mit dem Molmasse 142.18 (C₆H₇O₂S). MS (ESI): 143.0 (M+H⁺).

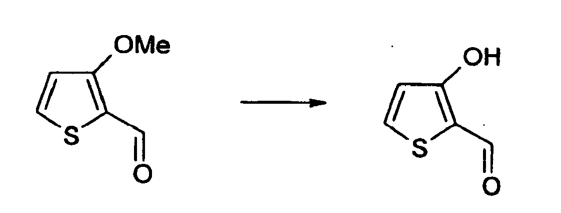

### b) 3-Hydroxy-thiophen-2-carbaldehyd

200 mg 3-Methoxy-thiophen-2-carbaldehyd werden in 5 ml Dichlormethan gelöst. 880 mg Bortribromid-Dimethylsulfid-Komplex werden in 5 ml Dichlormethan gelöst und zur Reaktionslösung gegeben. Die Lösung wird 18 h gerührt. Die Reaktionsmischung wird in 30 ml Wasser eingegossen und das Gemisch wird 4mal mit je 20 ml Dichlormethan extrahiert. Die vereinigten organischen Phasen werden mit 30 ml gesättigter Natriumchloridlösung gewaschen, über Natriumsulfat getrocknet und eingeengt. Man erhält 140 mg 3-Hydroxy-thiophen-2-carbaldehyd mit dem Molekulargewicht 128.15 (C₅H₄O₂S). MS (ESI): 129.0 (M+H⁺).

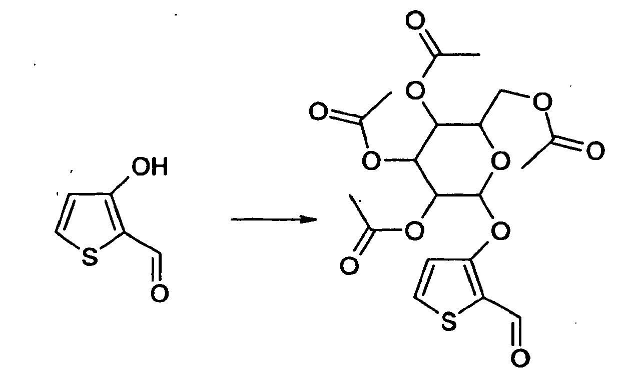

### c) Essigsäure-4,5-diacetoxy-6-acetoxymethyl-2-(2-formyl-thiophen-3-yloxy)-tetrahydropyran-3-yl-ester

3.81 g 3-Hydroxy-thiophen-2-carbaldehyd, 30,5 g Essigsäure-(4,5-diacetoxy-6-acetoxymethyl-2-[5-isopropyl-2-(4-methoxy-benzoyl)-thiophen-3-yloxy]-tetrahydropyran-3-yl)-ester, 37,0 g Kaliumcarbonat und 9,2 g Benzyltributylammoniumchlorid werden in 850 ml Dichlormethan gelöst. 7,5 ml Wasser werden zugegeben und die Reaktionsmischung wird 60h gerührt. Die Lösung wird mit Wasser und gesättigter Natriumchloridlösung extrahiert, die organische Phase wird über Natriumsulfat getrocknet und einrotiert. Zu dem erhaltenen bräunlichen Schaum werden 60 ml Ethanol:Wasser (9:1) gegeben und der entstehende feine Niederschlag wird abgesaugt. Man erhält das Produkt mit dem Molekulargewicht: 458,44 (C₁₉H₂₂O₁₁S), MS (ESI): 476 (M+NH₄⁺).

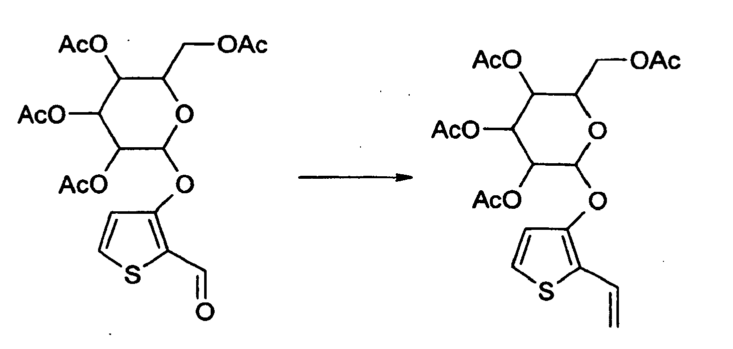

### d) Essigsäure-3,4,5-triacetoxy-6-(2-vinyl-thiophen-3-yloxy)-tetrahydropyran-2-ylmethylester

3.30 g Essigsäure-3,4,5-triacetoxy-6-(2-formyl-thiophen-3-yloxy)-tetrahydropyran-2-ylmethyl-ester werden in 60 ml Dioxan gelöst. 6.43 g Methyltriphenylphosphonium bromid, 5.37 g Kaliumcarbonat und 0,25 ml Wasser werden zugegeben und die Lösung wird 4 Std. lang refluxiert. Die Lösung wird eingeengt und durch Säulenfiltration gereinigt. Man erhält 2,89 g des Produkts mit dem Molekulargewicht: 456,47 (C₂₀H₂₄O₁₀S), MS (ESI): 479.10 (M+Na⁺); 474.10 (M+NH₄⁺).

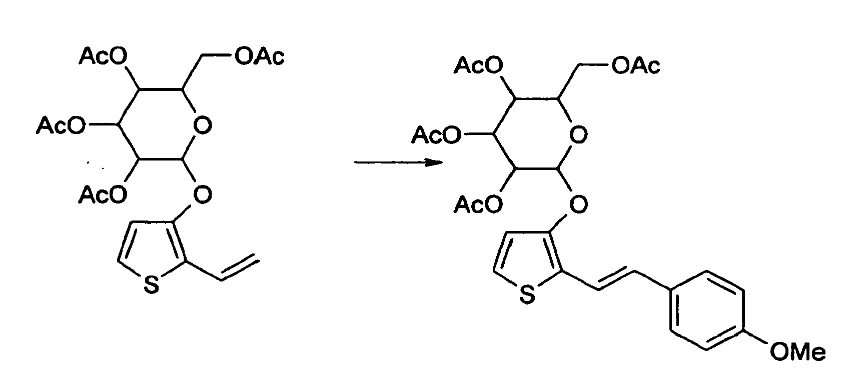

### e) Essigsäure-3,4,5-triacetoxy-6-{2-[2-(4-methoxy-phenyl)-vinyl]-thiophen-3-yloxy}-tetrahydropyran-2-ylmethyl-ester

148 mg Essigsäure-3,4,5-triacetoxy-6-(2-vinyl-thiophen-3-yloxy)-tetrahydropyran-2-ylmethyl-ester werden unter Argon in 2 ml Dichlormethan gelöst.
Tricyclohexylphosphin-[1,3-bis(2,4,6-trimethylphenyl)-4,5-dihydroimidazol-2-ylidene][benzyliden]ruthenium(IV)dichlorid (23 mg, gelöst in 2 ml Dichlormethan) wird zugegeben und die Lösung wird für 8 Std. unter Rückfluss erhitzt. Die Reaktionslösung wird eingeengt und säulenchromatographisch gereinigt (SiO₂, Heptan/ Ethylacetat 2:1). Man erhält 132 mg Produkt der Molmasse 562.60 (C₂₇H₃₀O₁₁S). MS(ESI): 575.20 (M+Na⁺).

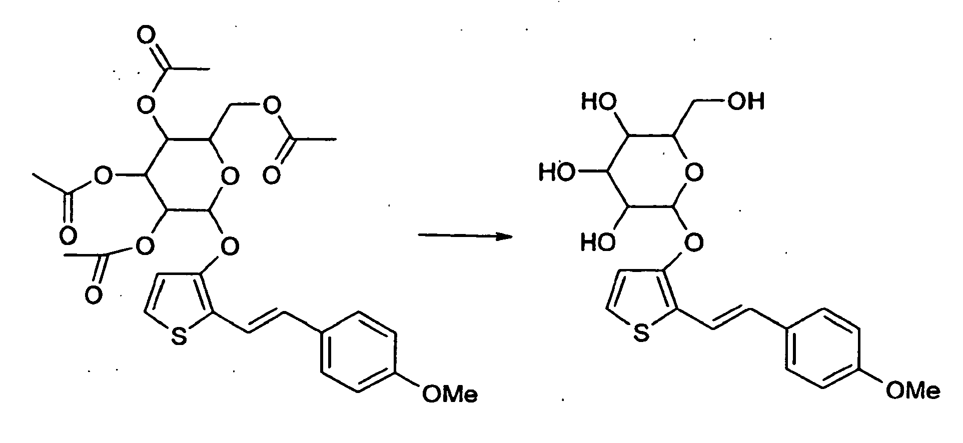

### f) 2-Hydroxymethyl-6-{2-[2 -(4-methoxy-phenyl)-vinyl]-thiophen-3-yloxy}-tetrahydropyran-3,4,5-triol = Beispiel 37

150 mg Essigsäure-3,4,5-triacetoxy-6-{2-[2-(4-methoxy-phenyl)-vinyl]-thiophen-3-yloxy}- tetrahydropyran-2-ylmethyl-ester werden in 10 ml trockenem Methanol suspendiert. 1.0 ml einer methanolischen NaOMe-Lösung (10 mg/ml) werden zugegeben. Die Lösung wird 18 Std. bei 22 °C gerührt. Amberlyst 15 (H⁺-Form) wird zugegeben und die Lösung wird mit 10 ml MeOH verdünnt, gefiltert und der Rückstand wird mit 20 ml Methanol gewaschen. Die organische Phase wird eingeengt und der Rückstand an Kieselgel chromatographisch gereinigt. Man erhält 100 mg des Produkts mit dem Molekulargewicht: 394,45 (C₁₉H₂₂O₇S), MS (ESI): 417 (M+Na⁺); 412 (M+NH₄⁺).

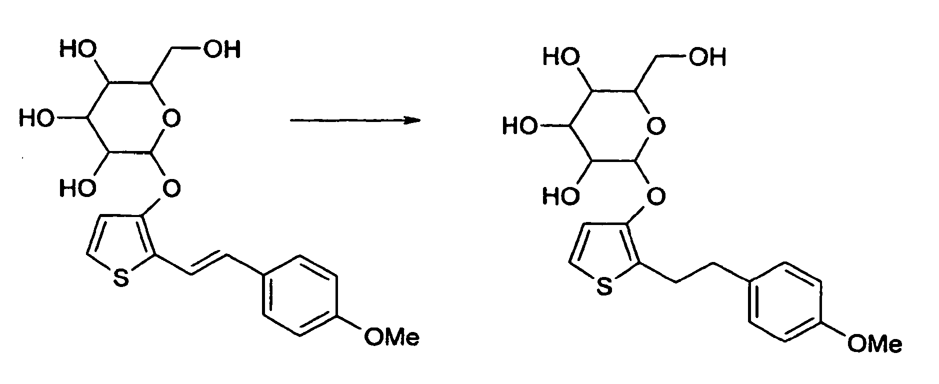

### g) 2-Hydroxymethyl-6-{2-[2-(4-methoxy-phenyl)-ethyl]-thiophen-3-yloxy}-tetrahydropyran-3,4,5-triol = Beispiel 36

50 mg 2-Hydroxymethyl-6-{2-[2-(4-methoxy-phenyl)-vinyl]-thiophen-3-yloxy}-tetrahydro-pyran-3,4,5-triol werden in 10 ml Methanol gelöst. 20 mg Palladium auf Aktivkohle werden zugegeben und die Lösung wird unter Wasserstoffatmosphäre für 18 Std. gerührt. Der Katalysator wird abfiltriert, mit 60ml Methanol gewaschen und die organische Phase wird eingeengt. Der Rückstand wird über Kieselgel chromatographiert (Essigsäureethylester). Man erhält 18 mg des Produkts mit dem Molekulargewicht 396,46 (C₁₉H₂₄O₇S); MS (ESI): 419,05 (M+Na⁺), 414,10 (M+NH₄⁺).

Auf dem gleichen Syntheseweg werden folgende Beispiel-Substanzen 38 bis 50 hergestellt.

| | | | |
|---|---|---|---|
| 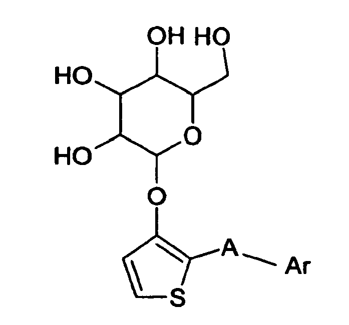 | | | |

| Beispiel | A | Ar | MS oder LC/MS |
|---|---|---|---|
| 38 | -CH=CH- | 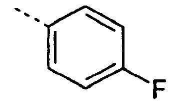 | OK |
| 39 | -CH=CH- | 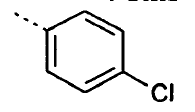 | OK |
| 40 | -CH=CH- | 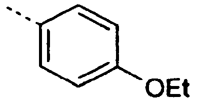 | OK |
| 41 | -CH=CH- | 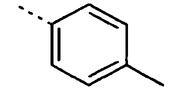 | OK |
| 42 | -CH=CH- | 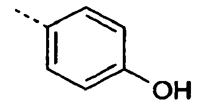 | OK |
| 43 | -CH₂-CH₂- | 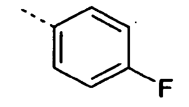 | OK |
| 44 | -CH₂-CH₂- | 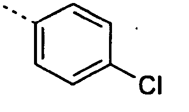 | OK |
| 45 | -CH₂-CH₂- | 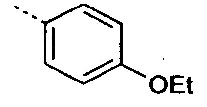 | OK |
| 46 | -CH₂-CH₂- | 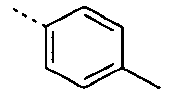 | OK |
| 47 | -CH₂-CH₂- | 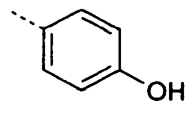 | OK |
| 48 | -CH=CH-CH₂- | 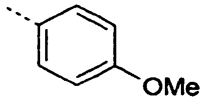 | OK |
| 49 | -CH₂-CH₂-CH₂- | 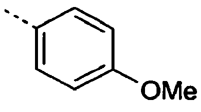 | OK |
| 50 | -CH=CH-CH₂- | 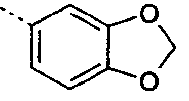 | OK |

Die Angabe MS/LCMS OK besagt, daß der Molpeak der angegebenen Verbindung als M+1 (MH⁺) und / oder als M+18 (MNH₄⁺) und / oder M + 23 (MNa⁺) erhalten wurde.

### Beispiel 51:

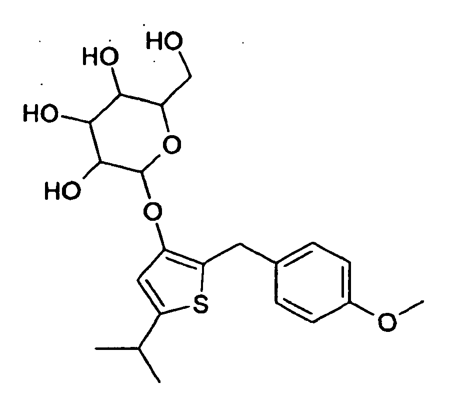

### 2-Hydroxymethyl-6-[5-isopropyl-2-(4-methoxy-benzyl)-thiophen-3-yloxy]-tetrahydropyran-3,4,5-triol

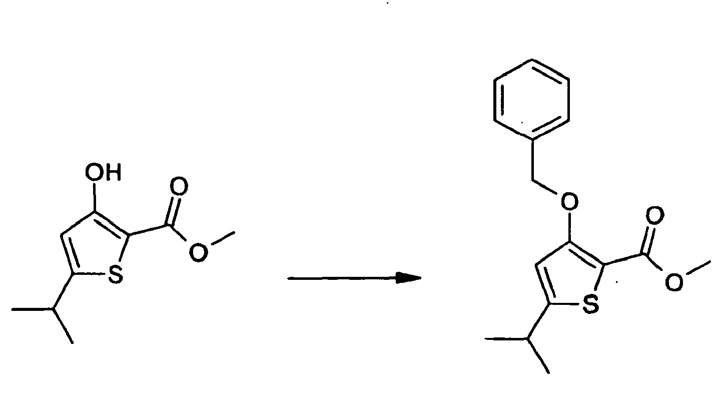

### a) 3-Benzyloxy-5-isopropyl-thiophen-2-carbonsäuremethylester

1.16 g 3-Hydroxy-5-isopropyl-thiophen-2-carbonsäuremethylester, die nach literaturbekanntem Verfahren synthetisiert wurden [H. Fiesselmann, F. Thoma, Chem. Ber. 1956, 89, 1907.], werden in 25 ml Dimethylformamid (DMF) gelöst und mit 2.83 g Cäsiumcarbonat und 1.72 ml Benzylbromid versetzt. Das Reaktionsgemisch wird 72 Std. bei 22 °C gerührt. Anschliessend werden 10 ml Methanol zugegeben und nach 30 Min. werden 100 ml gesättigte Natriumhydrogencarbonat-Lösung und 50 ml Wasser addiert. Die Mischung wird 3mal mit je 70 ml Diethylether extrahiert. Die vereinigten organischen Phasen werden über Natriumsulfat getrocknet und eingeengt. Das Rohprodukt wird durch Säulenchromatographie (SiO₂, Ethylacetat / n-Heptan = 1:4) gereinigt.
Man erhält das Produkt mit dem Molekulargewicht 290.4 (C₁₆H₁₈O₃S), MS (ESI): 291 (M+H⁺).

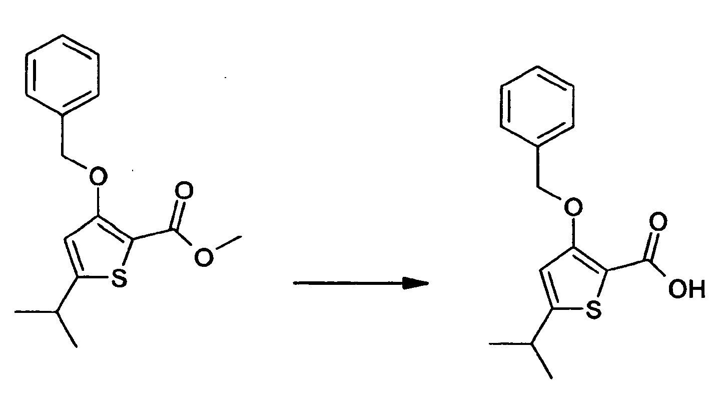

### b) 3-Benzyloxy-5-isopropyl-thiophen-2-carbonsäure

1.16 g 3-Benzyloxy-5-isopropyl-thiophen-2-carbonsäuremethylester werden in 10 ml Tetrahydrofuran (THF) und 10 ml Methanol gelöst und werden mit einer Lösung aus 1.7 g Lithiumhydroxid in 10 ml Wasser versetzt. Das Reaktionsgemisch wird 72 Std. bei 22 °C gerührt. Am Rotationsverdampfer werden Methanol und THF abgezogen. Unter Eiskühlung wird das Reaktionsgemisch mit 2-molarer Salzsäure auf pH = 4 eingestellt und 2mal mit je 50 ml Essigsäureethylester extrahiert. Die vereinigten organischen Phasen werden über Natriumsulfat getrocknet und eingeengt.
Man erhält das Produkt mit dem Molekulargewicht 276.4 (C₁₅H₁₈O₃S), MS (ESI): 294 (M+Na⁺).

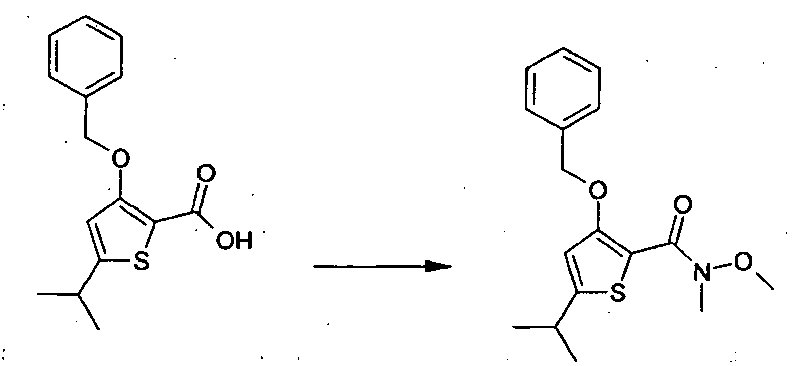

### c) 3-Benzyloxy-5-isopropyl-thiophen-2-carbonsäure-methoxy-methyl-amid

860 mg 3-Benzyloxy-5-isopropyl-thiophen-2-carbonsäure werden in 30 ml Dichlormethan gelöst und mit 560 mg N,O-Dimethylhydroxylamin Hydrochlorid und 2.3 ml Triethylamin versetzt. Nach 15 Min. bei 22 °C werden 2.3 ml einer 50%igen 1-Propanphosphonsäureanhydrid-Lösung in Essigsäure zugegeben und weitere 18 Std. bei 22 °C gerührt. Das Reaktionsgemisch wird zweimal mit je 70 ml Wasser und einmal mit 70 ml gesättigter Natriumchloridlösung gewaschen. Die organische Phase wird über Natriumsulfat getrocknet und eingeengt.
Man erhält das Produkt mit dem Molekulargewicht 319.4 (C₁₇H₂₁NO₃S), MS (ESI): 320 (M+H⁺).

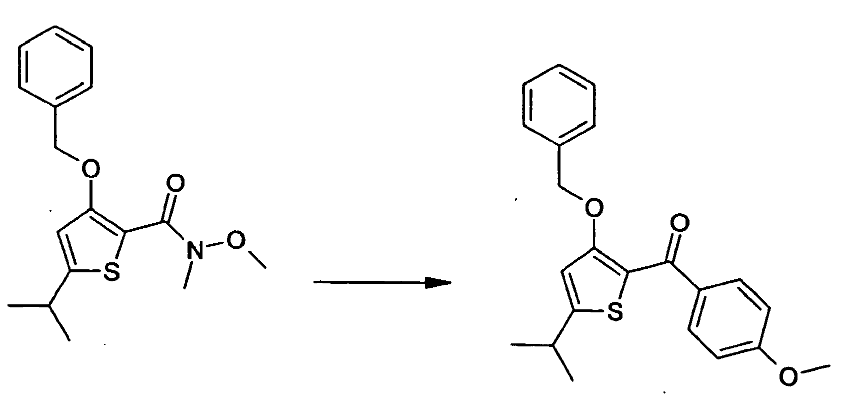

### d) (3-Benzyloxy-5-isopropyl-thiophen-2-yl)-(4-methoxy-phenyl)-methanon

860 mg 3-Benzyloxy-5-isopropyl-thiophen-2-carbonsäure-methoxy-methyl-amid werden in 50 ml Tetrahydrofuran (THF) gelöst, im Eisbad auf 0 °C gekühlt und mit 31.3 ml einer 0.5 molaren 4-Methoxyphenylmagnesiumbromid-Lösung in Tetrahydrofuran versetzt. Nach 30 Min. wird das Eisbad entfernt und das Reaktionsgemisch wird auf 22 °C aufgewärmt. Nach einer Std. wird das Reaktionsgemisch mit 70 ml gesättigter Natriumhydrogencarbonat-Lösung versetzt und 2mal mit je 100 ml Essigsäuremethylester extrahiert. Die vereinten organischen Phasen werden mit 70 ml gesättigter Natriumchloridlösung gewaschen, über Natriumsulfat getrocknet und eingeengt. Das Rohprodukt wird durch Säulenchromatographie (SiO₂, Ethylacetat / n-Heptan = 1:3) gereinigt.
Man erhält das Produkt mit dem Molekulargewicht 366.5 (C₂₂H₂₂O₃S), MS (ESI): 367 (m+H⁺).

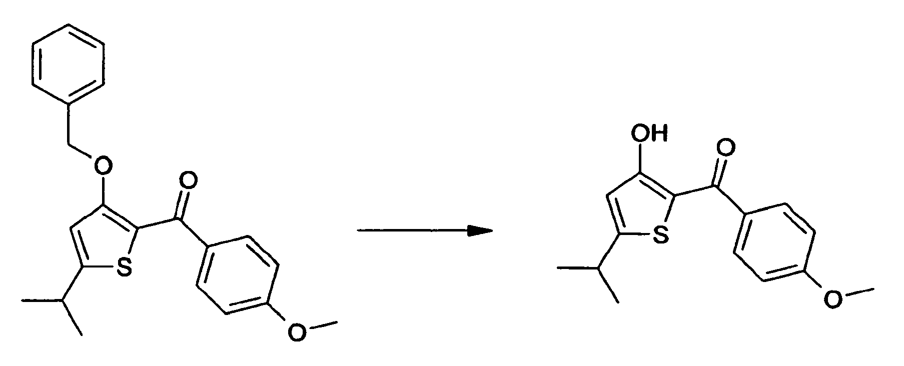

### e) (3-Hydroxy-5-isopropyl-thiophen-2-yl)-(4-methoxy-phenyl)-methanon

1.00 g (3-Benzyloxy-5-isopropyl-thiophen-2-yl)-(4-methoxy-phenyl)-methanon werden in 20 ml Dichlormethan gelöst. 2.73 ml einer 1 molaren Lösung aus Bortribromid-Dimethylsulfid-Komplex in Dichlormethan werden zur Reaktionslösung gegeben. Die Lösung wird 1.5 Std. bei 22 °C gerührt. Die Reaktionsmischung wird in 50 ml Wasser eingegossen und das Gemisch wird 2mal mit je 30 ml Dichlormethan extrahiert. Die vereinigte organische Phase wird 2mal mit je 30 ml gesättigter Natriumhydrogencarbonat-Lösung und einmal mit 50 ml gesättigter Natriumchlorid-Lösung extrahiert, über Natriumsulfat getrocknet und eingeengt. Das Rohprodukt wird durch Säulenchromatographie (SiO₂, Ethylacetat / n-Heptan = 1:4) gereinigt.
Man erhält das Produkt mit dem Molekulargewicht 276.4 (C₁₅H₁₆O₃S), MS (ESI): 299 (M+Na⁺).

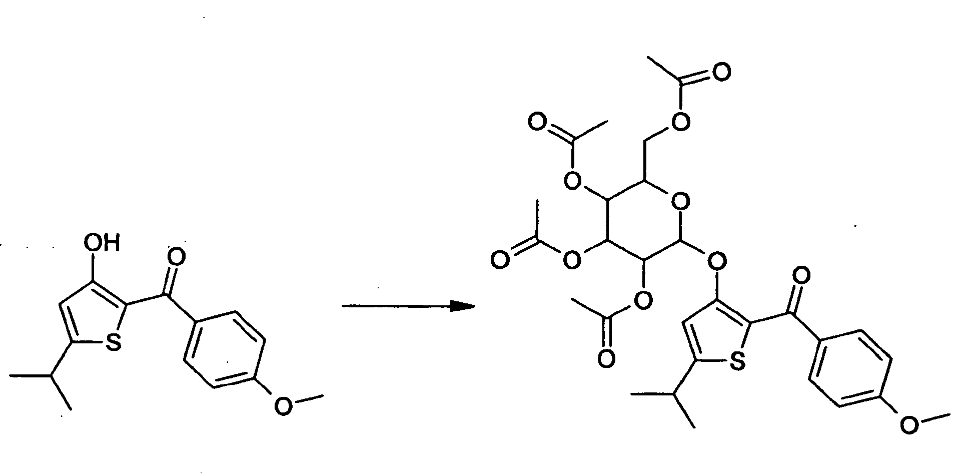

### f) Essigsäure-(4,5-diacetoxy-6-acetoxymethyl-2-[5-isopropyl-2-(4-methoxy-benzoyl)-thiophen-3- yloxy]-tetrahydro-pyran-3-yl)-ester

380 mg (3-Hydroxy-5-isopropyl-thiophen-2-yl)-(4-methoxy-phenyl)-methanon, 848 mg Essigsäure-4,5-diacetoxy-6-acetoxymethyl-2-bromo-tetrahydro-pyran-3-yl-ester, 1.43 g Kaliumcarbonat und 71.1 mg Benzyltributylammoniumchlorid werden in 20 ml Dichlormethan gelöst und es werden 1.20 ml Wasser zugegeben. Die Reaktionsmischung wird 40 Std. bei 22 °C gerührt. Das Reaktionsgemisch wird mit 50 ml Wasser versetzt und 2mal mit je 50 ml Dichlormethan extrahiert. Die vereinten organischen Phasen werden mit 50 ml gesättigter Natriumchlorid-Lösung gewaschen, über Natriumsulfat getrocknet und eingeengt. Das Rohprodukt wird durch Säulenchromatographie (SiO₂, Ethylacetat / n-Heptan = 1:1) gereinigt.
Man erhält das Produkt mit dem Molekulargewicht 606.7 (C₂₉H₃₄O₁₂S), MS (ESI): 607 (M+H⁺).

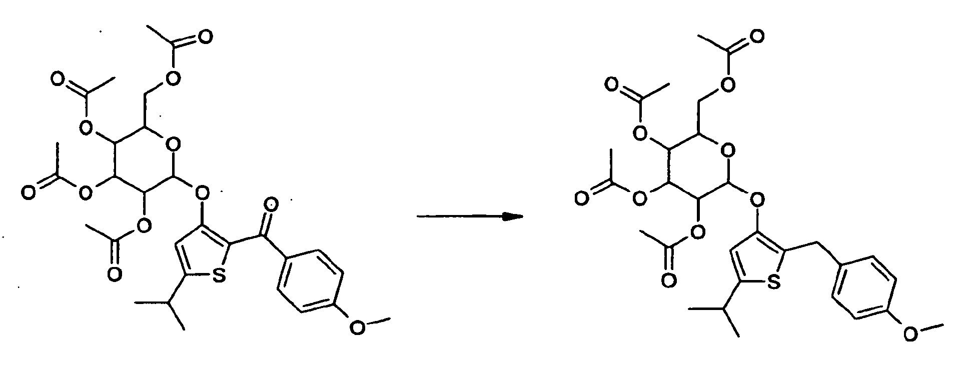

### g) Essigsäure-(4,5-diacetoxy-6-acetoxymethyl-2-[5-isopropyl-2-(4-methoxy-benzyl)-thiophen-3- yloxy]-tetrahydro-pyran-3-yl)-ester

630 mg Essigsäure-(4,5-diacetoxy-6-acetoxymethyl-2-[5-isopropyl-2-(4-methoxybenzoyl)-thiophen-3- yloxy]-tetrahydro-pyran-3-yl)-ester werden in 30 ml Acetonitril gelöst und im Eisbad auf 0 °C gekühlt. 1.31 ml Trimethylchlorosilan und 652 mg Natriumcyanoborhydrid werden zugegeben, das Eisbad wird entfernt und die Reaktion wird 2 Std. gerührt. Die Reaktionsmischung wird mit 100 ml Wasser versetzt und 2mal mit je 70 ml Dichlormethan extrahiert. Die vereinten organischen Phasen werden mit 50 ml gesättigter Natriumchloridlösung gewaschen, über Natriumsulfat getrocknet und eingeengt. Das Rohprodukt wird durch Säulenchromatographie (SiO₂, Ethylacetat / n-Heptan = 1:1) gereinigt.
Man erhält das Produkt mit dem Molekulargewicht 592.7 (C₂₉H₃₆O₁₁S), MS (ESI): 593 (M+H⁺).

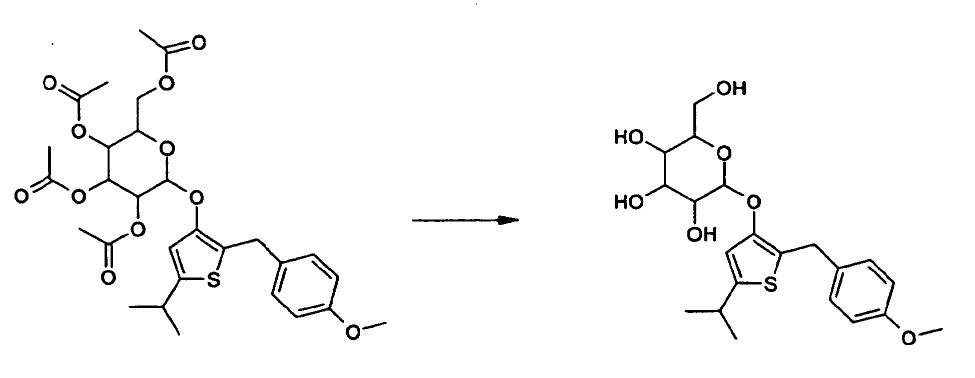

### h) 2-Hydroxymethyl-6-[5-isopropyl-2-(4-methoxy-benzyl)-thiophen-3-yloxy]-tetrahydropyran-3,4,5-triol

450 mg Essigsäure-(4,5-diacetoxy-6-acetoxymethyl-2-[5-isopropyl-2-(4-methoxybenzyl)-thiophen-3-yloxy]-tetrahydro-pyran-3-yl)-ester werden in 20 ml Methanol gelöst und mit 0.41 ml einer 30%igen methanolischen Natriummethanolat-Lösung versetzt. Das Reaktionsgemisch wird 1 Std. bei 22 °C gerührt, mit Amberlyst 15 (H⁺-Form) versetzt, filtriert und mit 30 ml Methanol nachgewaschen. Die Lösung wird eingeengt. Man erhält das Produkt mit dem Molekulargewicht 424.5 (C₂₁H₂₈O₇S), MS (ESI): 447 (M+Na⁺).

Auf dem gleichen Syntheseweg werden ausgehend von literaturbekannten 3-Hydroxythiophen-2-carbonsäuren [H. Fiesselmann, F. Thoma, Chem. Ber. 1956, 89, 1907-1913; M.D. Mullican et al. , J. Med. Chem. 1991, 34, 2186-2194; G.M. Karp et al., Synthesis 2000, 1078-1080.] die nachfolgenden Beispiele 52 bis 54 hergestellt:

| | | |
|---|---|---|
| 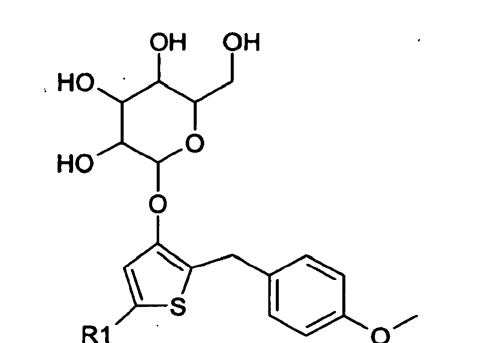 | | |

| **Beispiel** | **R₁** | **MS oder LC/MS** |
|---|---|---|
| **52** | 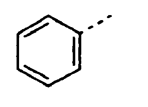 | **OK** |
| **53** | 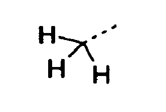 | **OK** |
| **54** | 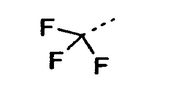 | **OK** |

### Beispiel 55:

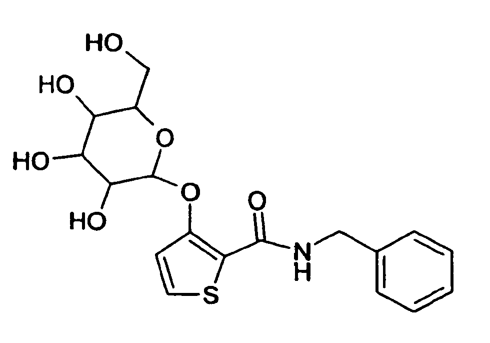

### 3-(3,4,5-Trihydroxy-6-hydroxymethyl-tetrahydro-pyran-2-yloxy)-thiophen-2-carbonsäure-benzylamid

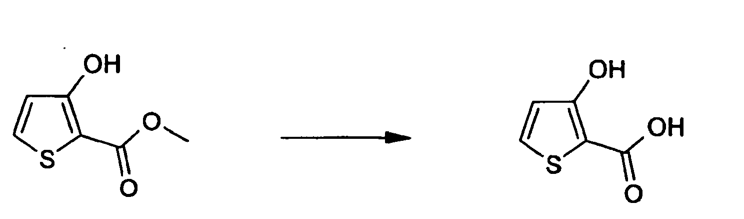

### a) 3-Hydroxy-thiophene-2-carbonsäure

10.0 g 3-Hydroxy-thiophene-2-carbonsäuremethylester werden in einer Mischung aus 90 ml Tetrahydrofuran (THF) und 90 ml Methanol gelöst und mit einer Lösung aus 25.2 g Lithiumhydroxid in 25 ml Wasser versetzt. Das Reaktionsgemisch wird 18 Std. bei 22 °C gerührt und anschliessend 6 Std. auf 55°C erwärmt. Das Reaktionsgemisch wird am Rotationsverdampfer auf 50 ml einggeengt, mit 2-molarer Salzsäure auf pH = 1 angesäuert und 3mal mit je 50 ml t-Butyl-methyl-ether extrahiert. Die vereinigten organischen Phasen werden über Magnesiumsulfat getrocknet und eingeengt.
Man erhält das Produkt mit dem Molekulargewicht 144.2 (C₅H₄O₃S), MS (ESI): 145 (m+H⁺).

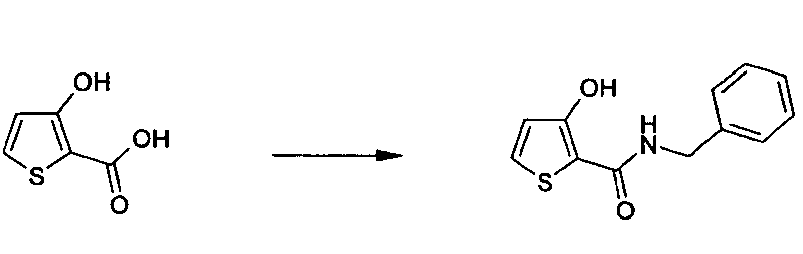

### b) 3-Hydroxy-thiophen-2-carbonsäurebenzylamid

1.44 g 3-Hydroxy-thiophene-2-carbonsäure werden in 100 ml Dichlormethan gelöst, mit 2.18 ml Benzylamin und 5.00 ml einer 50%igen 1-Propanphosphonsäureanhydrid-Lösung in Essigsäure versetzt. Das Reaktionsgemisch wird 2 Std. bei 22 °C gerührt und mit 100 ml gesättigter Natriumhydrogencarbonat-Lösung versetzt und 2mal mit je 100 ml Dichlormethan extrahiert. Die vereinten organischen Phasen werden mit 100 ml gesättigter Natriumchlorid-Lösung gewaschen, über Magnesiumsulfat getrocknet und eingeengt. Man erhält das Produkt mit dem Molekulargewicht 233.3 (C₁₂H₁₁NO₂S), MS (ESI): 234 (M+H⁺).

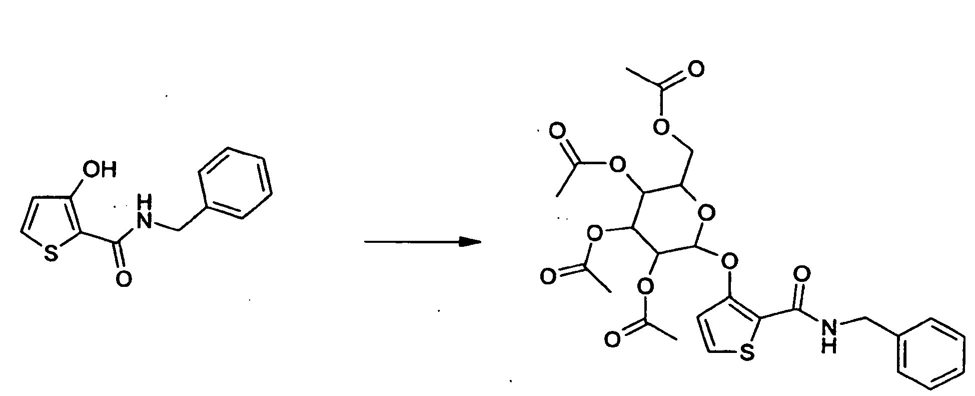

### c) Essigsäure-3,4,5-triacetoxy-6-(2-benzylcarbamoyl-thiophen-3-yloxy)-tetrahydropyran-2-ylmethylester

1.12 g 3-Hydroxy-thiophen-2-carbonsäurebenzylamid,-3.16 g Essigsäure-4,5-diacetoxy-6-acetoxymethyl-2-bromo-tetrahydro-pyran-3-yl-ester, 3.30 g Kaliumcarbonat und 235 mg Benzyltributylammoniumchlorid werden in 25 ml Dichlormethan gelöst und es werden 2.00 ml Wasser zugegeben. Die Reaktionsmischung wird 40 Std. bei 22 °C gerührt. Das Reaktionsgemisch wird mit 50 ml gesättigter Natriumhydrogencarbonat-Lösung versetzt und 2mal mit je 50 ml Dichlormethan extrahiert. Die vereinten organischen Phasen werden über Magnesiumsulfat getrocknet und eingeengt. Das Rohprodukt wird durch Säulenchromatographie (SiO₂, Ethylacetat / n-Heptan = 1:1) gereinigt.
Man erhält das Produkt mit dem Molekulargewicht 563.6 (C₂₆H₂₉NO₁₁S), MS (ESI): 564 (M+H⁺).

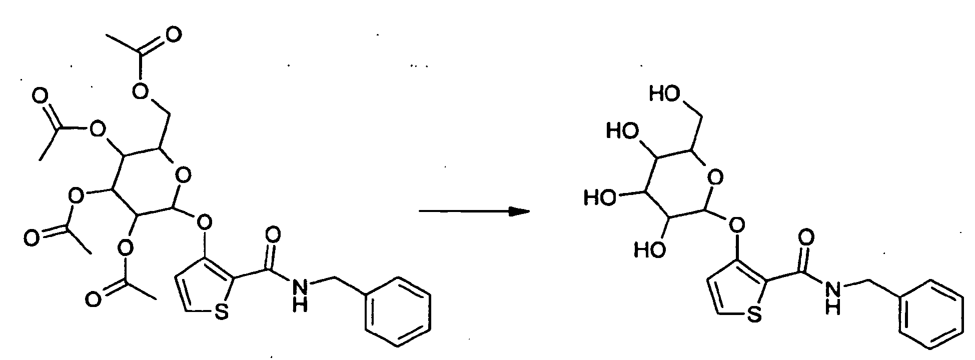

### d) 3-(3,4,5-Trihydroxy-6-hydroxymethyl-tetrahydro-pyran-2-yloxy)-thiophen-2-carbonsäure-benzylamid

600 mg Essigsäure-3,4,5-triacetoxy-6-(2-benzylcarbamoyl-thiophen-3-yloxy)-tetrahydro-pyran-2-ylmethylester werden in 40 ml Methanol gelöst und mit 1.40 ml einer 30%igen methanolischen Natriummethanolat-Lösung versetzt. Das Reaktionsgemisch wird 2 Std. bei 22 °C gerührt, mit 0.5-molarer methanolischer HCl-Lösung neutralisiert und eingeengt. Das Rohprodukt wird durch Säulenchromatographie (SiO₂, Ethylacetat / Methanol = 10:1) gereinigt.
Man erhält das Produkt mit dem Molekulargewicht 395.4 (C₁₈H₂₁NO₇S), MS (ESI): 396 (M+H⁺).

Auf dem gleichen Syntheseweg werden die nachfolgenden Beispiele 56 bis 58 hergestellt:

| Beispiel | A | Ar | MS/LCMS |
|---|---|---|---|
| 56 | -CO-NH-CH₂- | 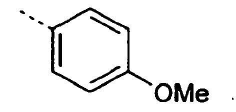 | OK |
| 57 | -CO-NH-CH₂- | 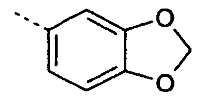 | OK |
| 58 | -CO-NH-CH₂- | 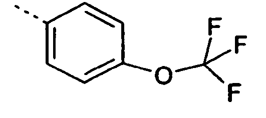 | OK |

## Patentansprüche

1. Zusammensetzung enthaltend eine Verbindung der Formel I 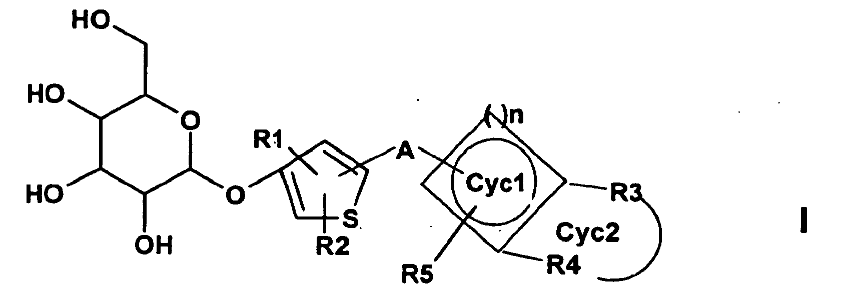 worin bedeuten
R1, R2 Wasserstoff, F, Cl, Br, J, OH, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]₂, (C₁- C₆)-Alkyl, (C₂-C₆)-Alkenyl, (C₂-C₆)-Alkinyl, (C₁-C₆)-Alkoxy, HO-(C₁-C₈)- Alkyl, (C₁-C₆)-Alkoxy-(C₁-C₆)-alkyl, Phenyl, Benzyl, (C₁-C₄)-Alkylcarbonyl, wobei in den Alkyl- und Alkoxyresten ein, mehrere, oder alle Wasserstoff(e) durch Fluor ersetzt sein können; SO₂-NH₂, SO₂NH(C₁-C₆)-Alkyl, SO₂N[(C₁-C₆)-Alkyl]₂, S-(C₁-C₆)-Alkyl, S- (CH₂)ₒ-Phenyl, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, (C₁-C₆)-Alkoxy, (C₁-C₆)- Alkyl, NH₂ substituiert sein kann; NH₂, NH-(C₁-C₆)-Alkyl, N((C₁-C₆)-Alkyl)₂, NH(C₁-C₇)-Acyl, Phenyl, O- (CH₂)ₒ-Phenyl, wobei o = 0 - 6 sein kann, wobei der Phenylring ein bis 3- fach substituiert sein kann mit F, Cl, Br, J, OH, CF₃, NO₂, CN, OCF₃, (C₁- C₆)-Alkoxy, (C₁-C₆)-Alkyl, NH₂, NH(C₁-C₆)-Alkyl, N((C₁-C₆)-Alkyl)₂, SO₂- CH₃, COOH, COO-(C₁-C₆)-Alkyl, CONH₂;
A (C₀-C₁₅)-Alkandiyl, wobei ein oder mehrere C-Atome des Alkandiylrests unabhängig voneinander durch -O-, =(C=O)-, -CH=CH-, -C≡C-, -S-, - CH(OH)-, -CHF-, -CF₂-, -(S=O)-, -(SO₂)-, -N((C₁-C₆)-Alkyl)-, -N((C₁-C₆)- Alkyl-Phenyl)- oder -NH- ersetzt sein können;
n eine Zahl von 0 bis 4;
Cyc1 ein 3 bis 7 gliedriger gesättigter, teilweise gesättigter oder ungesättigter Ring, wobei 1 C-Atom durch O oder S ersetzt sein kann;
R3, R4, R5 Wasserstoff, F, Cl, Br, J, OH, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂, (C₁- C₈)-Alkyl, (C₂-C₆)-Alkenyl, (C₂-C₆)-Alkinyl, (C₁-C₁₂)-Alkoxy, HO-(C₁-C₆)- Alkyl, (C₁-C₆)-Alkoxy-(C₁-C₆)-alkyl, wobei in den Alkyl- und Alkoxyresten ein, mehrere, oder alle Wasserstoff(e) durch Fluor ersetzt sein können; SO₂-NH₂, SO₂NH(C₁-C₆)-Alkyl, SO₂N[(C₁-C₆)-Alkyl]₂, S-(C₁-C₆)-Alkyl, S- (CH₂)ₒ-Phenyl, SO-(C₁-C₆)-Alkyl, SO-(CH₂)ₒ-Phenyl, SO₂-(C₁-C₆)-Alkyl, SO₂-(CH₂)ₒ-Phenyl, wobei o = 0 - 6 sein kann und der Phenylrest bis zu zweifach mit F, Cl, Br, OH, CF₃, NO₂, CN, OCF₃, (C₁-C₆)-Alkoxy, (C₁-C₆)- Alkyl, NH₂ substituiert sein kann; NH₂, NH-(C₁-C₆)-Alkyl, N((C₁-C₆)-Alkyl)₂, NH(C₁-C₇)-Acyl, Phenyl, (CH₂)ₒ- Phenyl, O-(CH₂)ₒ-Phenyl, wobei o = 0 - 6 sein kann, wobei der Phenylring ein bis 3-fach substituiert sein kann mit F, Cl, Br, J, OH, CF₃, NO₂, CN, OCF₃, (C₁-C₈)-Alkoxy, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂; oder
R3 und R4 gemeinsam mit den sie tragenden C-Atomen einen 5 bis 7 gliedrigen, gesättigten, teilweise oder vollständig ungesättigten Ring Cyc2, wobei 1 oder 2 C-Atom(e) des Ringes auch durch N, O oder S ersetzt sein können und Cyc2 gegebenenfalls durch (C₁-C₆)-Alkyl, (C₂-C₅)-Alkenyl, (C₂-C₅)- Alkinyl, wobei jeweils eine CH₂-Gruppe durch O ersetzt sein kann, oder
R5 durch F, Cl, OH, CF₃, NO₂, CN, COO(C₁-C₄)-Alkyl, CONH₂, CONH(C₁- C₄)-Alkyl, OCF₃ substituiert sein kann und Wasserstoff;
sowie deren pharmazeutisch verträglichen Salze
und einen weiteren Wirkstoff, ausgewählt aus der Gruppe der Biguanide oder der Sulfonylharnstoffe.

2. Zusammensetzung gemäß Anspruch 1, worin A mit dem Thienylring in 2 Position verknüpft ist.

3. Zusammensetzung gemäß Anspruch 1 oder 2, worin bedeuten
R1, R2 Wasserstoff, F, Cl, Br, J, OH, NO₂, CN, COOH, CO(C₁-C₆)-Alkyl, COO(C₁-C₆)-Alkyl, CONH₂, CONH(C₁-C₆)-Alkyl, CON[(C₁-C₆)-Alkyl]₂, (C₁- C₆)-Alkyl, (C₂-C₆)-Alkenyl, (C₂-C₆)-Alkinyl, (C₁-C₆)-Alkoxy, HO-(C₁-C₆)- Alkyl, (C₁-C₆)-Alkoxy-(C₁-C₆)-alkyl, Phenyl, Benzyl, (C₁-C₄)-Alkylcarbonyl, wobei in den Alkyl- und Alkoxyresten ein, mehrere, oder alle Wasserstoff(e) durch Fluor ersetzt sein können; SO-(C₁-C₆)-Alkyl;
A (C₀-C₁₅)-Alkandiyl, wobei ein oder mehrere C-Atom(e) des Alkandiylrests unabhängig voneinander durch -O-, -(C=O)-, -CH=CH-, -C≡C-, -S-, - CH(OH)-, -CHF-, -CF₂-, -(S=O)-, -(SO₂)-, -N((C₁-C₆)-Alkyl)-, -N((C₁-C₆)- Alkyl-Phenyl)- oder -NH- ersetzt sein können;
n eine Zahl 2 oder 3;
Cyc1 ein 5 bis 6 gliedriger gesättigter, teilweise gesättigter oder ungesättigter Ring, wobei 1 C-Atom durch O oder S ersetzt sein kann;
R3, R4, R5 Wasserstoff, F, Cl, Br, J, OH, NO₂, CN, COOH, COO(C₁-C₆)-Alkyl, CO(C₁-C₄)-Alkyl, CONH₂, CONH(C₁-C₆)-Alkyl, CON[(C₁-C₆)-Alkyl]₂, (C₁- C₈)-Alkyl, (C₂-C₆)-Alkenyl, (C₂-C₆)-Alkinyl, (C₁-C₁₂)-Alkoxy, HO-(C₁-C₆)- Alkyl, (C₁-C₆)-Alkoxy-(C₁-C₆)-alkyl, wobei in den Alkyl- und Alkxoyresten ein, mehrere, oder alle Wasserstoff(e) durch Fluor ersetzt sein können; (CH₂)ₒ-Phenyl, O-(CH₂)ₒ-Phenyl, wobei o = 1 - 4 sein kann, S-(C₁-C₆)- Alkyl, SO-(C₁-C₆)-Alkyl; oder
R3 und R4 gemeinsam mit den sie tragenden C-Atomen einen 5 bis 7 gliedrigen, gesättigten, teilweise oder vollständig ungesättigten Ring Cyc2, wobei 1 oder 2 C-Atom(e) des Ringes auch durch N, O oder S ersetzt sein können und Cyc2 gegebenenfalls durch (C₁-C₆)-Alkyl, (C₂-C₅)-Alkenyl, (C₂-C₅)- Alkinyl, wobei jeweils eine CH₂-Gruppe durch O ersetzt sein kann, oder durch F, Cl, OH, CF₃, NO₂, CN, COO(C₁-C₄)-Alkyl, CONH₂, CONH(C₁- C₄)-Alkyl, OCF₃ substituiert sein kann, und
R5 Wasserstoff.

4. Zusammensetzung gemäß den Ansprüchen 1 bis 3, worin
R1, R2 Wasserstoff, (C₁-C₆)-Alkyl, (C₁-C₄)-Alkoxy, HO-(C₁-C₄)-Alkyl, (C₁-C₄)- Alkoxy-(C₁-C₄)-alkyl, F, Cl, CF₃, OCF₃ , OCH₂CF₃ (C₁-C₄)-Alkyl-CF₂-, Phenyl, Benzyl, (C₁-C₄)-Alkylcarbonyl, (C₂-C₄)-Alkenyl, (C₂-C₄)-Alkinyl, COO(C₁-C₄)-Alkyl;
A -CH=CH-CH₂- oder (C₁-C₄)-Alkandiyl, wobei eine oder zwei CH₂-Gruppen auch ersetzt sein können durch -(C=O)-, -CH=CH-, -CH(OH)-, -NH-, - CHF-, -CF₂-, -O-_{;}
n eine Zahl 2 oder 3;
Cyc1 ungesättigter Ring, wobei 1 C-Atom durch O oder S ersetzt sein kann;
R3,R4,R5 Wasserstoff, F, Cl, Br, J, NO₂, OH, CN, (C₁-C₆)-Alkyl, (C₁-C₈)-Alkoxy, OCF₃, OCH₂CF₃, S-(C₁-C₄)-Alkyl, COOH, HO-(C₁-C₄)-Alkyl, (C₁-C₄)- Alkoxy-(C₁-C₄)-alkyl, (CH₂)ₒ-Phenyl, O-(CH₂)ₒ-Phenyl, wobei o = 1 - 2 sein kann, oder
R3 und R4 gemeinsam -CH=CH-O-, -CH=CH-S-, -O-(CH₂)ₚ-O-, mit p = 1 oder 2, -O- CF₂-O-, -CH=CH-CH=CH- und
R5 Wasserstoff bedeuten.

5. Zusammensetzung gemäß den Ansprüchen 1 bis 4, worin R2 Wasserstoff bedeutet.

6. Zusammensetzung gemäß den Ansprüchen 1 bis 5, worin
R1 Wasserstoff, CF₃, (C₁-C₄)-Alkyl, Phenyl,
R2 Wasserstoff,
A -CH₂-, -C₂H₄-, -C₃H₆, -CH(OH)-, -(C=O)-, -CH=CH-, -CH=CH-CH₂-, - CO-CH₂-CH₂- oder -CO-NH-CH₂-;
n eine Zahl 2 oder 3;
Cyc1 ungesättigter Ring, wobei 1 C-Atom durch S ersetzt sein kann;
R3,R4,R5 Wasserstoff, F, Cl, J, NO₂, OH, CN, (C₁-C₆)-Alkyl, (C₁-C₈)-Alkoxy, O-CH₂- Phenyl, OCF₃, S-CH₃, COOH oder
R3 und R4 gemeinsam -CH=CH-O-, -O-(CH₂)p-O-, mit p = 1 oder 2, -O-CF₂-O-, - CH=CH-CH=CH-, und
R5 Wasserstoff bedeuten.

7. Zusammensetzung gemäß den Ansprüchen 1 bis 6, worin
A -CH₂- oder -CH₂-CH₂- bedeutet.

8. Zusammensetzung gemäß den Ansprüchen 1 bis 7, worin
Cyc1 Phenyl bedeutet.

9. Zusammensetzung gemäß den Ansprüchen 1 bis 7, worin
Cyc1 Thienyl bedeutet.

10. Zusammensetzung gemäß den Ansprüchen 1 bis 8, worin
Cyc1 monosubstituiert ist.

11. Zusammensetzung gemäß Anspruch 1, enthaltend eine Verbindung, ausgewählt aus der Gruppe: 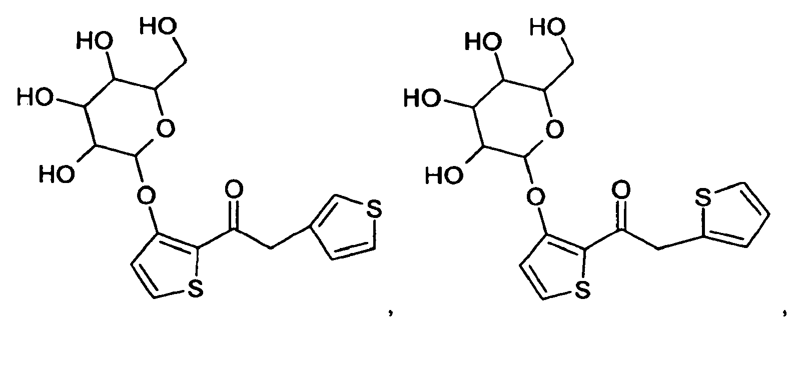 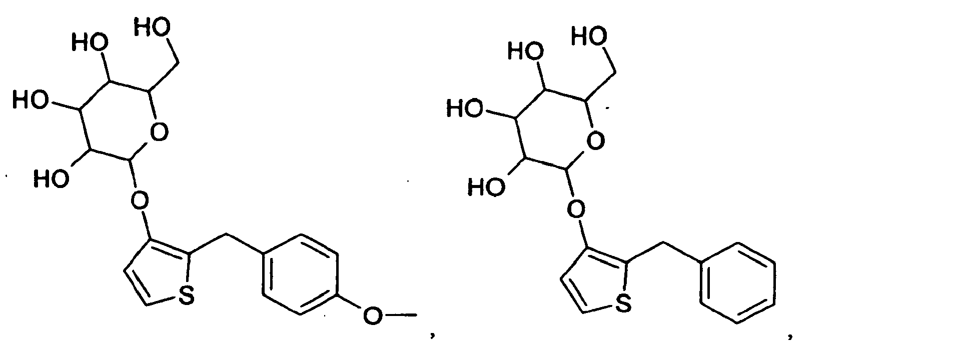 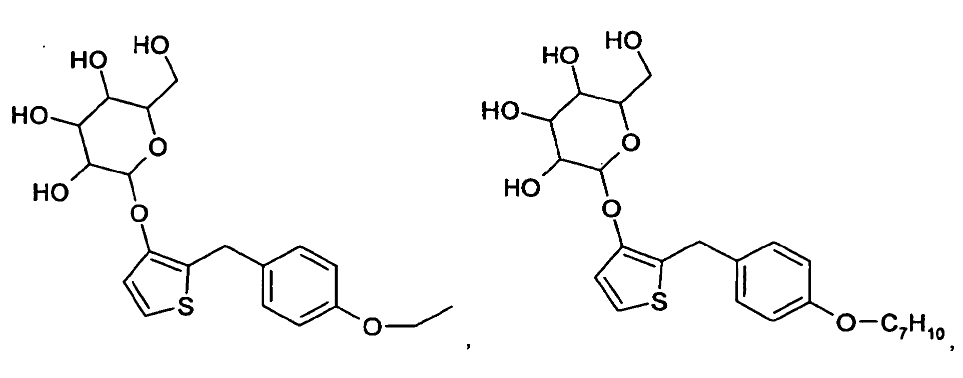 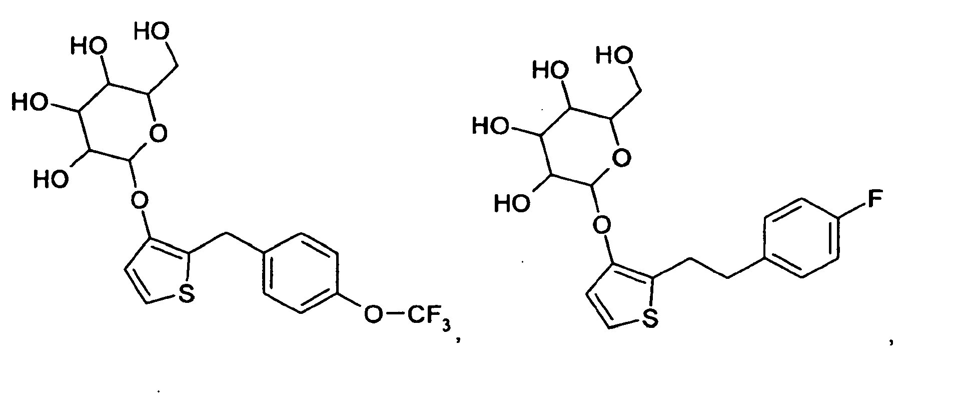 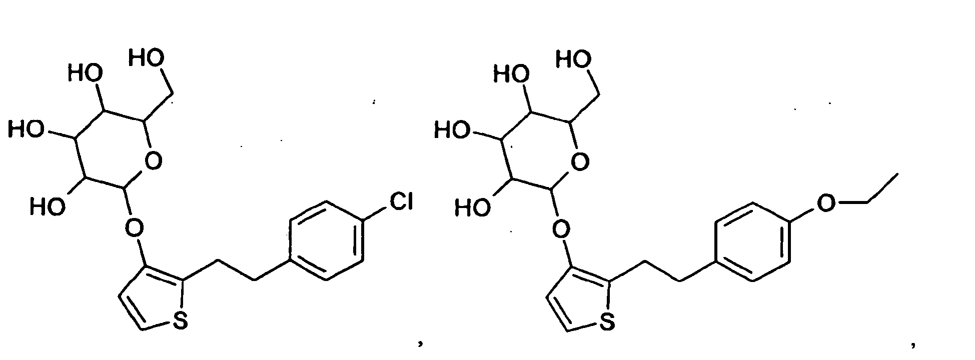 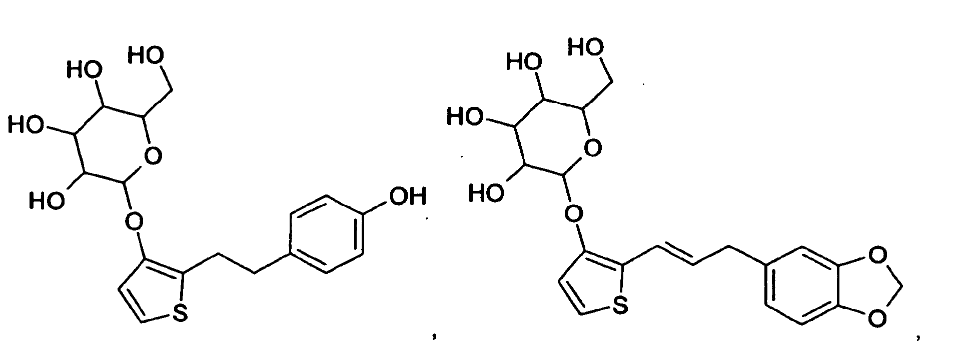 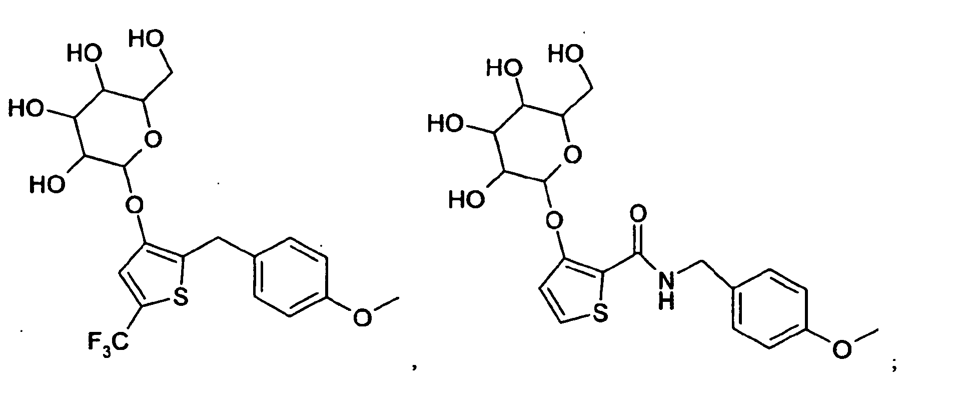 in ihrer β-D-Gluco-Form.

12. zusammensetzung gemäß Anspruch 1, enthaltend eine Verbindung, ausgewählt aus der Gruppe: 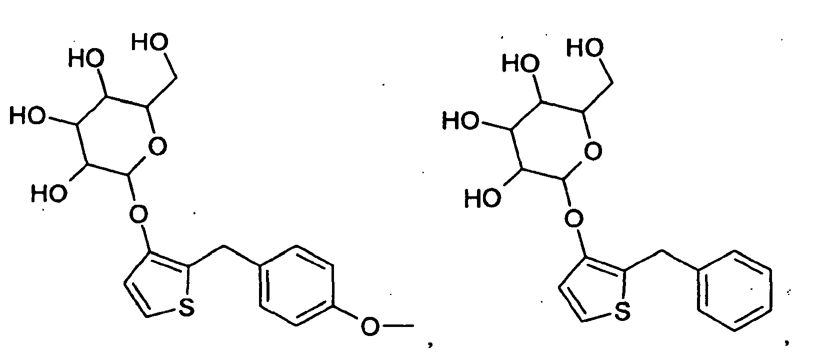 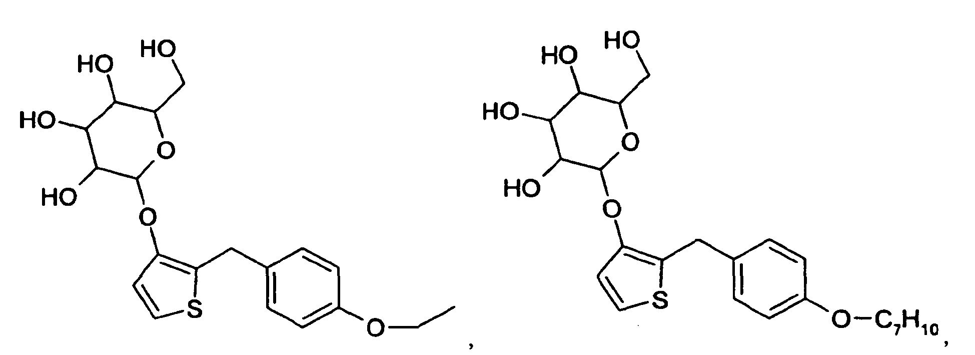 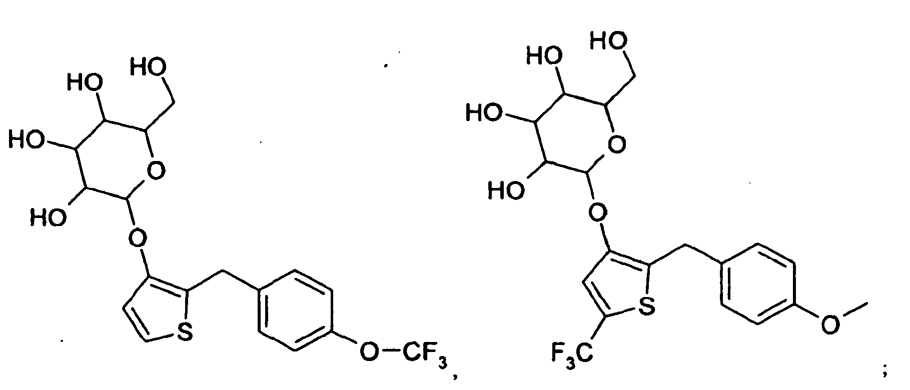 in ihrer β-D-Gluco-Form.

13. Zusammensetzung gemäß Ansprüchen 1 bis 12, **dadurch gekennzeichnet, dass** das Biguanid Metformin ist.

14. Zusammensetzung gemäß Ansprüchen 1 bis 12, **dadurch gekennzeichnet, dass** der Sulfonylharnstoff Tolbutamid, Glibenclamid, Glipizid oder Glimepirid ist.

15. Arzneimittel enthaltend eine Zusammensetzung gemäß einem oder mehreren der Ansprüche 1 bis 14.

16. Verwendung der Zusammensetzung gemäß einem oder mehreren der Ansprüche 1 bis 14 zur Herstellung eines Medikamentes zur Behandlung des Typ 1 und Typ 2 Diabetes.

17. Verwendung der Zusammensetzung gemäß einem oder mehreren der Ansprüche 1 bis 14 zur Herstellung eines Medikamentes zur Blutzuckersenkung.

18. Verwendung einer Verbindung der Formel I gemäß einem oder mehreren der Ansprüche 1 bis 12 in Kombination mit Metformin zur Herstellung eines Medikaments zur Behandlung des Typ 1 und Typ 2 Diabetes.

19. Verwendung einer Verbindung der Formel I gemäß einem oder mehreren der Ansprüche 1 bis 12 in Kombination mit Metformin zur Herstellung eines Medikaments zur Blutzuckersenkung.

20. Verwendung einer Verbindung der Formel I gemäß einem oder mehreren der Ansprüche 1 bis 12 in Kombination mit einem Sulfonylharnstoff ausgewählt aus der Gruppe Tolbutamid, Glibenclamid, Glipizid und Glimepirid zur Herstellung eines Medikaments zur Behandlung des Typ 1 und Typ 2 Diabetes.

21. Verwendung einer Verbindung der Formel I gemäß einem oder mehreren der Ansprüche 1 bis 12 in Kombination mit einem Sulfonylharnstoff ausgewählt aus der Gruppe Tolbutamid, Glibenclamid, Glipizid und Glimepirid zur Herstellung eines Medikaments zur Blutzuckersenkung.

22. Verwendung einer Verbindung der Formel I gemäß einem oder mehreren der Ansprüche 1 bis 12 in Kombination mit Insulin zur Herstellung eines Medikaments zur Behandlung des Typ 1 und Typ 2 Diabetes.

23. Verwendung einer Verbindung der Formel I gemäß einem oder mehreren der Ansprüche 1 bis 12 in Kombination mit Insulin zur Herstellung eines Medikaments zur Blutzuckersenkung.

## Claims

1. Composition comprising a compound of the formula I 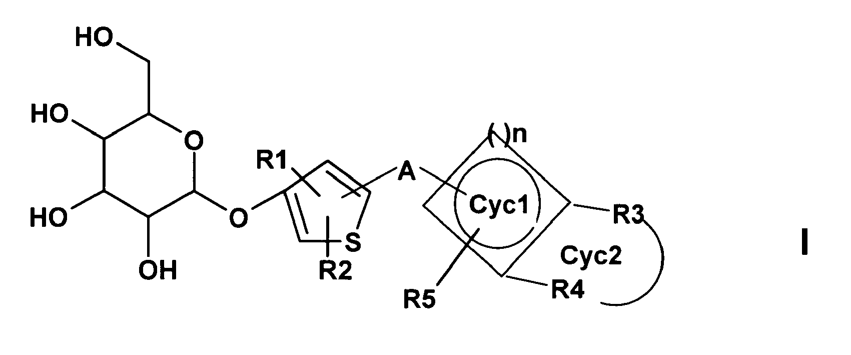 in which
R1, R2 are hydrogen, F, Cl, Br, I, OH, NO₂, CN, COOH, CO(C₁-C₆)-alkyl, COO(C₁-C₆)-alkyl, CONH₂, CONH(C₁-C₆)-alkyl, CON[(C₁-C₆)-alkyl]₂, (C₁-C₆)- alkyl, (C₂-C₆)-alkenyl, (C₂-C₆)-alkynyl, (C₁-C₆)- alkoxy, HO-(C₁-C₈)-alkyl, (C₁-C₆)-alkoxy-(C₁-C₆)- alkyl, phenyl, benzyl, (C₁-C₄)-alkylcarbonyl, where one, more than one or all hydrogen(s) in the alkyl and alkoxy radicals may be replaced by fluorine; SO₂-NH₂, SO₂NH(C₁-C₆)-alkyl, SO₂N[(C₁-C₆)-alkyl]₂, S-(C₁-C₆)-alkyl, S- (CH₂)ₒ-phenyl, SO-(C₁-C₆)- alkyl, SO-(CH₂)ₒ-phenyl, SO₂-(C₁-C₆)-alkyl, SO₂- (CH₂)ₒ-phenyl, where o may be 0-6 and the phenyl radical may be substituted up to twice by F, Cl, Br, OH, CF₃, NO₂, CN, OCF₃, (C₁-C₆)-alkoxy, (C₁-C₆)-alkyl, NH₂; NH₂, NH-(C₁-C₆) -alkyl, N ((C₁-C₆)-alkyl)₂, NH(C₁-C₇)-acyl, phenyl, O-(CH₂)ₒ-phenyl, where o may be 0-6 and where the phenyl ring may be substituted one to 3 times by F, Cl, Br, I, OH, CF₃, NO₂, CN, OCF₃, (C₁-C₆) -alkoxy, (C₁-C₆) -alkyl, NH₂, NH(C₁-C₆)-alkyl, N((C₁-C₆)-alkyl)₂, SO₂-CH₃, COOH, COO-(C₁-C₆)-alkyl, CONH₂;
A is (C₀-C₁₅) -alkanediyl, where one or more carbon atoms in the alkanediyl radical may be replaced independently of one another by -O-, -(C=O)-, -CH=CH-, -C≡C-, -S-, -CH(OH)-, -CHF-, -CF₂-, -(S=O)-, -(SO₂)-, -N((C₁-C₆)-alkyl) -, -N((C₁-C₆)- alkylphenyl)- or -NH-;
n is a number from 0 to 4;
Cyc1 is a 3- to 7-membered, saturated, partially saturated or unsaturated ring, where 1 carbon atom may be replaced by O or S;
R3, R4, R5 are hydrogen, F, Cl, Br, I, OH, NO₂, CN, COOH, COO(C₁-C₆)-alkyl, CO(C₁-C₄)-alkyl, CONH₂, CONH (C₁-C₆)-alkyl, CON [(C₁-C₆)-alkyl]₂, (C₁-C₈)- alkyl, (C₂-C₆)-alkenyl, (C₂-C₆)-alkynyl, (C₁-C₁₂)- alkoxy, HO-(C₁-C₆)-alkyl, (C₁-C₆)-alkoxy-(C₁-C₆)- alkyl, where one, more than one or all hydrogen(s) in the alkyl and alkoxy radicals may be replaced by fluorine; SO₂-NH₂, SO₂NH(C₁-C₆)-alkyl, SO₂N[(C₁-C₆)-alkyl]₂, S-(C₁-C₆)-alkyl, S-(CH₂)ₒ-phenyl, SO-(C₁-C₆)- alkyl, SO-(CH₂)ₒ-phenyl, SO₂-(C₁-C₆)-alkyl, SO₂- (CH₂)ₒ-phenyl, where o may be 0-6 and the phenyl radical may be substituted up to twice by F, Cl, Br, OH, CF₃, NO₂, CN, OCF₃, (C₁-C₆) -alkoxy, (C₁-C₆)-alkyl, NH₂; NH₂, NH-(C₁-C₆)-alkyl, N((C₁-C₆)-alkyl)₂, NH(C₁-C₇)-acyl, phenyl, (CH₂)ₒ-phenyl, O-(CH₂)ₒ- phenyl, where o may be 0-6 and where the phenyl ring may be substituted one to 3 times by F, Cl, Br, I, OH, CF₃, NO₂, CN, OCF₃, (C₁-C₈)- alkoxy, (C₁-C₆)-alkyl, NH₂, NH(C₁-C₆)-alkyl, N((C₁-C₆)-alkyl)₂, SO₂-CH₃, COOH, COO-(C₁-C₆)- alkyl, CONH₂; or
R3 and R4 together with the carbon atoms carrying them are a 5- to 7-membered, saturated, partially or completely unsaturated ring Cyc2, where 1 or 2 carbon atom(s) in the ring may also be replaced by N, O or S, and Cyc2 may optionally be substituted by (C₁-C₆)-alkyl, (C₂-C₅)-alkenyl, (C₂-C₅)-alkynyl, where in each case one CH₂ group may be replaced by 0, or substituted by F, Cl, OH, CF₃, NO₂, CN, COO(C₁-C₄)-alkyl, CONH₂, CONH(C₁-C₄)-alkyl, OCF₃, and
R5 is hydrogen;
and the pharmaceutically acceptable salts thereof and a further active ingredient selected from the group of biguanides or sulfonylureas.

2. Composition according to Claim 1, in which A is linked to the thienyl ring in position 2.

3. Composition according to Claim 1 or 2, in which
R1, R2 are hydrogen, F, Cl, Br, I, OH, NO₂, CN, COOH, CO(C₁-C₆) -alkyl, COO (C₁-C₆) -alkyl, CONH₂, CONH (C₁-C₆) -alkyl, CON[(C₁-C₆) -alkyl]₂, (C₁-C₆)- alkyl, (C₂-C₆) -alkenyl, (C₂-C₆)-alkynyl, (C₁-C₆)- alkoxy, HO-(C₁-C₆)-alkyl, (C₁-C₆)-alkoxy-(C₁-C₆)- alkyl, phenyl, benzyl, (C₁-C₄)-alkylcarbonyl, where one, more than one or all hydrogen(s) in the alkyl and alkoxy radicals may be replaced by fluorine; SO-(C₁-C₆)-alkyl;
A is (C₀-C₁₅)-alkanediyl, where one or more carbon atom(s) in the alkanediyl radical may be replaced independently of one another by -0-, -(C=O) -CH=CH-, -C≡C-, -S-, -CH(OH)-, -CHF-, -CF₂-, -(S=O)-, -(SO₂)-, -N((C₁-C₆)-alkyl)-, -N((C₁-C₆)-alkylphenyl)- or -NH-;
n is a number 2 or 3;
Cyc1 is a 5- to 6-membered, saturated, partially saturated or unsaturated ring, where 1 carbon atom may be replaced by 0 or S;
R3, R4, R5 are hydrogen, F, Cl, Br, I, OH, NO₂, CN, COOH, COO(C₁-C₆)-alkyl, CO(C₁-C₄)-alkyl, CONH₂, CONH(C₁-C₆)-alkyl, CON[(C₁-C₆)-alkyl]₂, (C₁-C₈)- alkyl, (C₂-C₆)-alkenyl, (C₂-C₆)-alkynyl, (C₁-C₁₂)- alkoxy, HO-(C₁-C₆)-alkyl, (C₁-C₆)-alkoxy-(C₁-C₆)- alkyl, where one, more than one or all hydrogen(s) in the alkyl and alkoxy radicals may be replaced by fluorine; (CH₂)ₒ-phenyl, O-(CH₂)ₒ-phenyl, where o may be 1-4, S-(C₁-C₆)- alkyl, SO-(C₁-C₆)-alkyl; or
R3 and R4 together with the carbon atoms carrying them are a 5- to 7-membered, saturated, partially or completely unsaturated ring Cyc2, where 1 or 2 carbon atom(s) in the ring may also be replaced by N, O or S, and Cyc2 may optionally be substituted by (C₁-C₆)-alkyl, (C₂-C₅)-alkenyl, (C₂-C₅)-alkynyl, where in each case one CH₂ group may be replaced by 0, or substituted by F, Cl, OH, CF₃, NO₂, CN, COO(C₁-C₄)-alkyl, CONH₂, CONH(C₁-C₄)-alkyl, OCF₃, and
R5 is hydrogen.

4. Composition according to Claims 1 to 3, in which
R1, R2 are hydrogen, (C₁-C₆)-alkyl, (C₁-C₄)-alkoxy, HO- (C₁-C₄)-alkyl, (C₁-C₄)-alkoxy-(C₁-C₄)-alkyl, F, Cl, CF₃, OCF₃, OCH₂CF₃ (C₁-C₄)-alkyl-CF₂-, phenyl, benzyl, (C₁-C₄)-alkylcarbonyl, (C₂-C₄)-alkenyl, (C₂-C₄)-alkynyl, COO(C₁-C₄)-alkyl;
A is -CH=CH-CH₂- or (C₁-C₄)-alkanediyl, where one or two CH₂ groups may also be replaced by -(C=O)-, -CH=CH-, -CH(OH)-, -NH-, -CHF-, -CF₂-, -O-;
n is a number 2 or 3;
Cyc1 is unsaturated ring, where 1 carbon atom may be replaced by O or S;
R3, R4, R5 are hydrogen, F, Cl, Br, I, NO₂, OH, CN, (C₁-C₆)-alkyl, (C₁-C₈)-alkoxy, OCF₃, OCH₂CF₃, S-(C₁-C₄)-alkyl, COOH, HO-(C₁-C₄)-alkyl, (C₁-C₄)- alkoxy- (C₁-C₄)-alkyl, (CH₂)ₒ-phenyl, O- (CH₂)ₒ- phenyl, where o may be 1-2 or
R3 and R4 together are -CH=CH-O-, -CH=CH-S-, -O-(CH₂)ₚ- 0-, with p = 1 or 2, -O-CF₂-O-, -CH=CH-CH=CH-, and
R5 is hydrogen.

5. Composition according to Claims 1 to 4, in which R2 is hydrogen.

6. Composition according to Claims 1 to 5, in which
R1 is hydrogen, CF₃, (C₁-C₄)-alkyl, phenyl,
R2 is hydrogen,
A is -CH₂-, -C₂H₄-, -C₃H₆, -CH(OH)-, -(C=O)-, -CH=CH-, -CH=CH-CH₂-, -CO-CH₂-CH₂- or -CO-NH- CH₂-;
n is a number 2 or 3;
Cyc1 is unsaturated ring, where 1 carbon atom may be replaced by S;
R3, R4, R5 are hydrogen, F, Cl, I, NO₂, OH, CN, (C₁-C₆)- alkyl, (C₁-C₈)-alkoxy, O-CH₂-phenyl, OCF₃, S-CH₃, COOH or
R3 and R4 together are -CH=CH-O-, -O-(CH₂)ₚ-O-, with p = 1 or 2, -O-CF₂-O-, -CH=CH-CH=CH-, and
R5 is hydrogen.

7. Composition according to Claims 1 to 6, in which
A is -CH₂- or -CH₂-CH₂-.

8. Composition according to Claims 1 to 7, in which
Cyc1 is phenyl.

9. Composition according to Claims 1 to 7, in which
Cyc1 is thienyl.

10. Composition according to Claims 1 to 8, in which
Cyc1 is monosubstituted.

11. Composition according to Claim 1, containing a compound selected from the group: 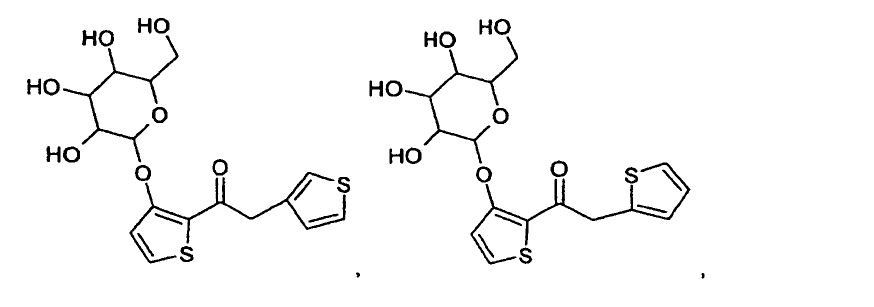 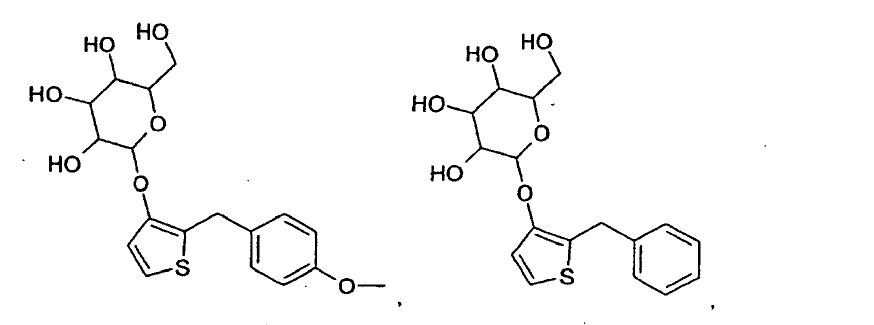 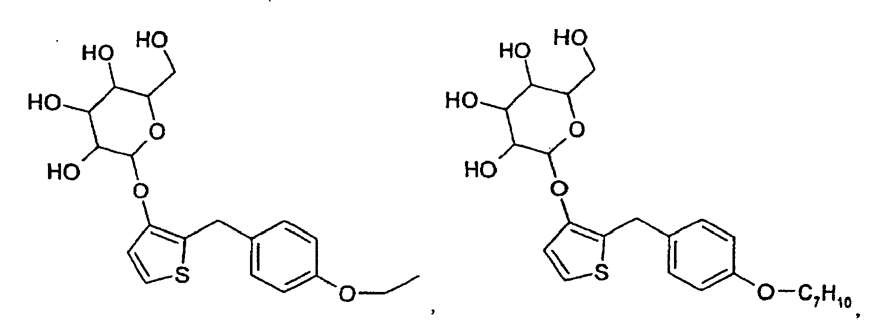 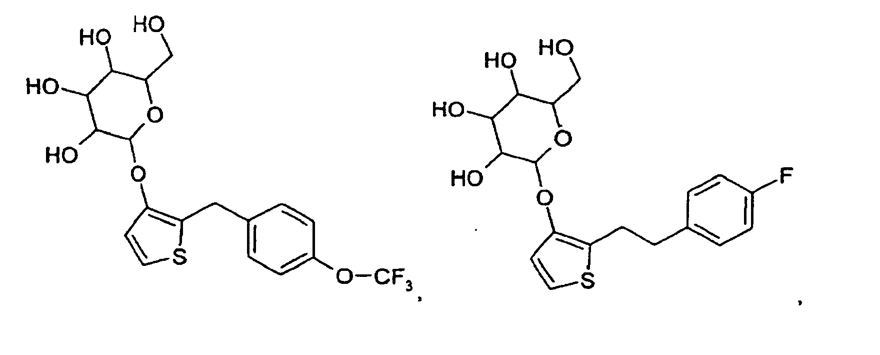 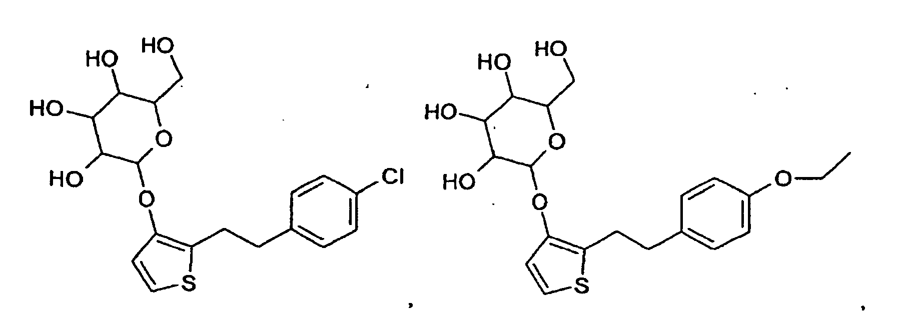 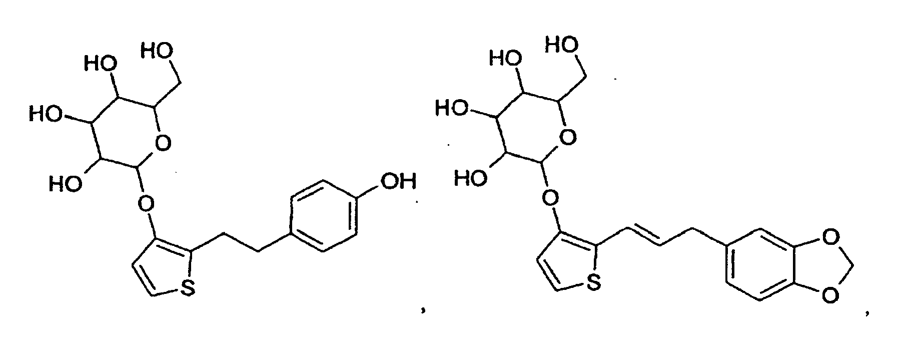 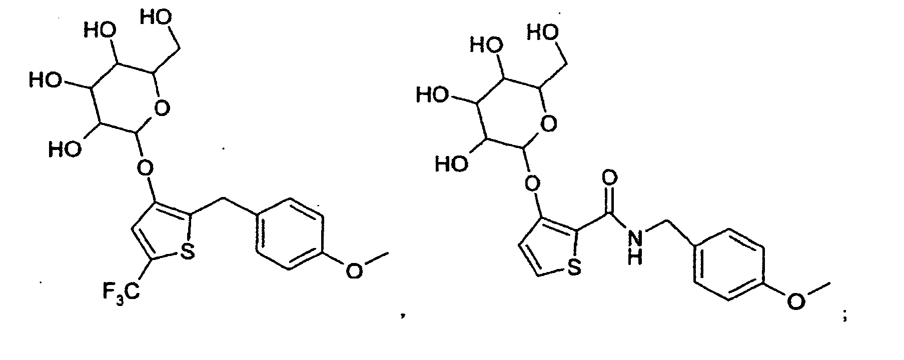 in their β-D-gluco form.

12. Composition according to Claim 1, containing a compound selected from the group: 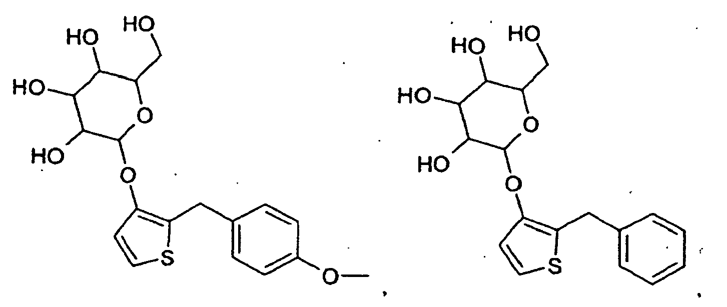 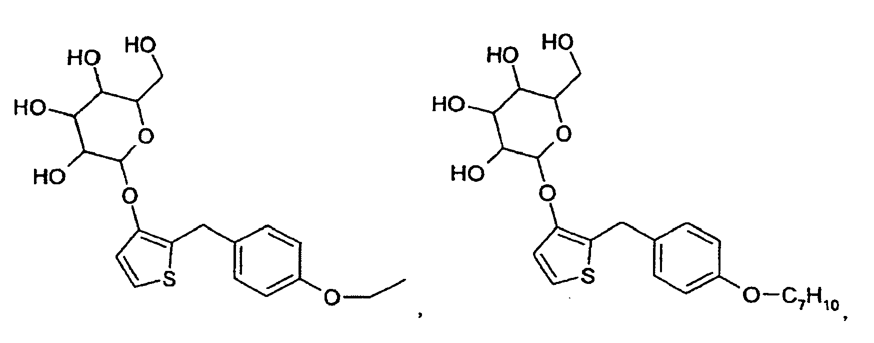 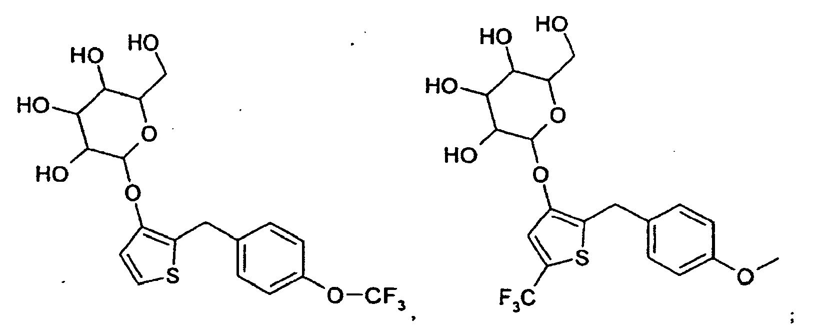 in their β-D-gluco form.

13. Composition according to Claims 1 to 12, wherein the biguanide is metformin.

14. Composition according to Claims 1 to 12, wherein the sulfonylurea is tolbutamide, glibenclamide, glipizide or glimepiride.

15. Medicament comprising a composition according to one or more of Claims 1 to 14.

16. Use of the composition according to one or more of Claims 1 to 14 for producing a medicament for the treatment of type 1 and type 2 diabetes.

17. Use of the composition according to one or more of Claims 1 to 14 for producing a medicament for lowering blood glucose.

18. Use of a compound of the formula I according to one or more of Claims 1 to 12 in combination with metformin for producing a medicament for the treatment of type 1 and type 2 diabetes.

19. Use of a compound of the formula I according to one or more of Claims 1 to 12 in combination with metformin for producing a medicament for lowering blood glucose.

20. Use of a compound of the formula I according to one or more of Claims 1 to 12 in combination with a sulfonylurea selected from the group consisting of tolbutamide, glibenclamide, glipizide and glimepiride for producing a medicament for the treatment of type 1 and type 2 diabetes.

21. Use of a compound of the formula I according to one or more of Claims 1 to 12 in combination with a sulfonylurea selected from the group consisting of tolbutamide, glibenclamide, glipizide and glimepiride for producing a medicament for lowering blood glucose.

22. Use of a compound of the formula I according to one or more of Claims 1 to 12 in combination with insulin for producing a medicament for the treatment of type 1 and type 2 diabetes.

23. Use of a compound of the formula I according to one or more of Claims 1 to 12 in combination with insulin for producing a medicament for lowering blood glucose.

## Revendications

1. Composition comprenant un composé de formule I, 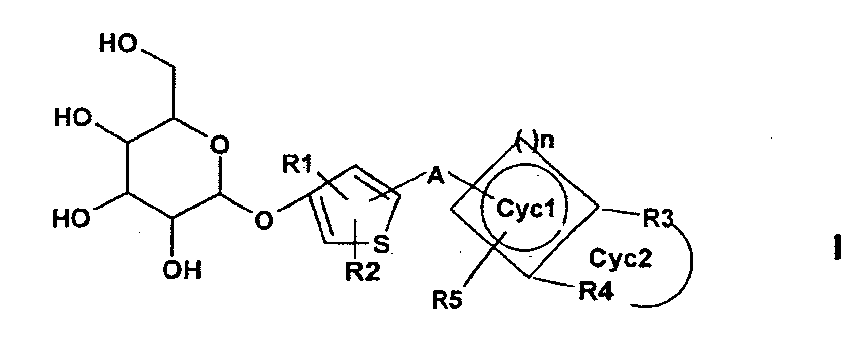 dans laquelle
R1, R2 représentent un atome d'hydrogène ou de F, Cl, Br, I ou un groupe OH, NO₂, CN, COOH, CO-alkyle(C₁-C₆), COO-alkyle (C₁-C₆), CONH₂, CONH-alkyle(C₁-C₆), CON [alkyle (C₁-C₆)]₂, alkyle en C₁-C₆, alcényle en C₂-C₆, alcynyle en C₂-C₆, alcoxy en C₁-C₆, HO-alkyle (C₁-C₈), alcoxy(C₁-C₆)-alkyle(C₁-C₆), phényle, benzyle, alkyl(C₁-C₄)-carbonyle, un, plusieurs ou tous les atome(s) d'hydrogène dans les radicaux alkyle et alcoxy pouvant être remplacé(s) par le fluor ; SO₂-NH₂, SO₂NH-alkyle(C₁-C₆), SO₂N [alkyle (C₁-C₆)]₂, S-alkyle (C₁-C₆), S-(CH₂)ₒ-phényle, SO-alkyle(C₁-C₆), SO-(CH₂)ₒ-phényle, SO₂-alkyle(C₁-C₆), SO₂-(CH2)ₒ-phényle, o pouvant être 0-6 et le cycle phényle pouvant être substitué jusqu'à deux fois par un ou des atomes de F, Cl, Br ou groupes OH, CF₃, NO₂, CN, OCF₃, alcoxy en C₁-C₆, alkyle en C₁-C₆, NH₂ ; NH₂, NH-alkyle(C₁-C₆), N [alkyle (C₁-C₆)]₂, NH[acyle(C₁-C₇)], phényle, O-(CH₂)ₒ-phényle, o pouvant être 0-6, le cycle phényle pouvant être substitué jusqu'à 3 fois par un ou des atomes de F, Cl, Br, I ou groupes OH, CF₃, NO₂, CN, OCF₃, alcoxy en C₁-C₆, alkyle en C₁-C₆, NH₂, NH-alkyle (C₁-C₆), N [alkyle (C₁-C₆)]₂, SO₂-CH₃, COOH, COO-alkyle(C₁-C₆), CONH₂ ;
A représente un radical alcane (C₀-C₁₅) diyle, un ou plusieurs atomes de carbone du radical alcanediyle pouvant être remplacés, chacun indépendamment, par -O-, -(C=O)-, -CH=CH-, -C≡C-, -S-, -CH(OH)-, -CHF-, -CF₂-, -(S=O)-, -(SO₂)-, -N[alkyl(C₁-C₆)]--N[alkyl (C₁-C₆)- phényle]- ou -NH- ;
n est un nombre allant de 0 à 4 ;
Cyc1 représente un cycle saturé, partiellement saturé ou insaturé à 3- 7 chaînons, 1 atome de carbone pouvant être remplacé par 0 ou S ;
R3, R4, R5 représentent un atome d'hydrogène ou de F, Cl, Br, I ou un groupe OH, NO₂, CN, COOH, COO-alkyle(C₁-C₆), CO-alkyle(C₁-C₄), CONH₂, CONH-alkyle(C₁-C₆), CON[alkyle(C₁-C₆)]₂, alkyle en C₁-C₈, alcényle en C₂-C₆, alcynyle en C₂-C₆, alcoxy en C₁-C₁₂, HO-alkyle(C₁-C₆), alcoxy(C₁-C₆)-alkyle(C₁-C₆), un, plusieurs ou tous les atome(s) d'hydrogène dans les radicaux alkyle et alcoxy pouvant être remplacé(s) par le fluor ; SO₂-NH₂, SO₂NH-alkyle(C₁-C₆), SO₂N [alkyle(C₁-C₆)]₂, S-alkyle(C₁-C₆), S-(CH₂)ₒ-phényle, SO-alkyle(C₁-C₆), SO-(CH₂)ₒ-phényle, SO₂-alkyle(C₁-C₆), SO₂-(CH₂)ₒ-phényle, o pouvant être 0-6 et le radical phényle pouvant être substitué jusqu'à deux fois par un ou des atomes de F, Cl, Br ou groupes OH, CF₃, NO₂, CN, OCF₃, alcoxy en C₁-C₆, alkyle en C₁-C₆, NH₂ ; NH₂, NH-alkyle(C₁-C₆), N [alkyle (C₁-C₆)]₂, NH[acyle(C₁-C₇)], phényle, (CH₂)ₒ-phényle, 0- (CH₂)ₒ-phényle, o pouvant être 0-6, le radical phényle pouvant être substitué jusqu'à 3 fois par un ou des atomes de F, Cl, Br, I ou groupes OH, CF₃, NO₂, CN, OCF₃, alcoxy en C₁-C₈, alkyle en C₁-C₆, NH₂, NH-alkyle(C₁-C₆), N [alkyle (C₁-C₆)]₂, SO₂-CH₃, COOH, COO-alkyle(C₁-C₆), CONH₂ ; ou
R3 et R4 représentent ensemble, avec les atomes de carbone qui les portent, un cycle. Cyc2 saturé, partiellement ou totalement insaturé, à 5-7 chaînons, 1 ou 2 atomes de carbone du cycle pouvant également être remplacés par N, 0 ou S, et Cyc2 pouvant éventuellement être substitué par un groupe alkyle en C₁-C₆, alcényle en C₂-C₅, alcynyle en C₂-C₅, un groupe CH₂ dans chaque cas pouvant être remplacé par 0, ou pouvant être substitué par F, Cl, OH, CF₃, NO₂, CN, COO- alkyle(C₁-C₄), CONH₂, CONH-alkyle(C₁-C₄), OCF₃ et
R5 représente un atome d'hydrogène ;
ainsi que leurs sels pharmaceutiquement acceptables et au moins une autre substance active choisi parmi le groupe constitué par les biguanides et les sulfonylurées.

2. Composition selon la revendication 1, dans laquelle A est lié au cycle thiényle en position 2.

3. Composition selon la revendication 1 ou 2, dans laquelle
R1, R2 représentent un atome d'hydrogène ou de F, Cl, Br, I ou un groupe OH, NO₂, CN, COOH, CO-alkyle(C₁-C₆), COO-alkyle(C₁-C₆), CONH₂, CONH-alkyle(C₁-C₆), CON[alkyle(C₁-C₆)]₂, alkyle en C₁-C₆, alcényle en C₂-C₆, alcynyle en C₂-C₆, alcoxy en C₁-C₆, HO-alkyle(C₁-C₆), alcoxy(C₁-C₆)-alkyle(C₁-C₆), phényle, benzyle, alkyl(C₁-C₄)-carbonyle, un, plusieurs ou tous les atome(s) d'hydrogène dans les radicaux alkyle et alcoxy pouvant être remplacé(s) par le fluor ; SO-alkyle(C₁-C₆) ;
A représente un radical alcane(C₀- C₁₅)diyle, un ou plusieurs atomes de carbone du radical alcanediyle pouvant être remplacés, chacun indépendamment, par -O-, -(C=O)-, -CH=CH-, -C≡C-, -S-, -CH(OH)-, -CHF-, -CF₂-, -(S=O)-, - (SO₂)-, -N[alkyl(C₁-C₆)]-, -N[alkyl(C₁-C₆)-phényle]- ou -NH- ;
n est un nombre égal à 2 ou 3 ;
Cyc1 représente un cycle saturé, partiellement saturé ou insaturé à 5- 6 chaînons, 1 atome de carbone pouvant être remplacé par 0 ou S ;
R3, R4, R5 représentent un atome d'hydrogène ou de F, Cl, Br, I ou un groupe OH, NO₂, CN, COOH, COO-alkyle(C₁-C₆), CO-alkyle(C₁-C₄), CONH₂, CONH-alkyle(C₁-C₆), CON[alkyle(C₁-C₆)]₂. alkyle en C₁-C₈, alcényle en C₂-C₆, alcynyle en C₂-C₆, alcoxy en C₁-C₁₂, HO-alkyle(C₁-C₆), alcoxy(C₁-C₆)-alkyle(C₁-C₆), un, plusieurs ou tous les atome(s) d'hydrogène dans les radicaux alkyle et alcoxy pouvant être remplacé(s) par le fluor ; (CH₂)ₒ-phényle, O-(CH₂)ₒ-phényle, o pouvant être 1-4, S-alkyle(C₁-C₆), SO-alkyle(C₁-C₆) ; ou
R3 et R4 représentent ensemble, avec les atomes de carbone qui les portent, un cycle Cyc2 saturé, partiellement ou totalement insaturé, à 5-7 chaînons, 1 ou 2 atomes de carbone du cycle pouvant également être remplacés par N, 0 ou S, et Cyc2 pouvant éventuellement être substitué par un groupe alkyle en C₁-C₆, alcényle en C₂-C₅, alcynyle en C₂-C₅, un groupe CH₂ dans chaque cas pouvant être remplacé par O, ou pouvant être substitué par F, Cl, OH, CF₃, NO₂, CN, COO-alkyle(C₁-C₄), CONH₂, CONH-alkyle(C₁-C₄), OCF₃ et
R5 représente un atome d'hydrogène.

4. Composition selon les revendications 1 à 3, dans laquelle
R1, R2 représentent un atome d'hydrogène ou un groupe alkyle en C₁-C₆, alcoxy en C₁-C₄, HO-alkyle(C₁-C₄), alcoxy(C₁-C₄)-alkyle(C₁-C₄), F, Cl, un groupe CF₃, OCF₃, OCH₂CF₃, alkyl(C₁-C₄)-CF₂, phényle, benzyle, alkyl(C₁-C₄)-carbonyle, alcényle en C₂-C₄, alcynyle en C₂-C₄, COO- alkyle(C₁-C₄) ;
A représente -CH=CH-CH₂- ou un radical alcane(C₁-C₄)diyle, un ou deux groupes CH₂ pouvant également être remplacés par -(C=O)-, -CH=CH-, -CH(OH)-, -NH-, -CHF-, -CF₂-, -O- ;
n est un nombre égal à 2 ou 3 ;
Cyc1 représente un cycle insaturé, 1 atome de carbone pouvant être remplacé par O ou S ;
R3, R4, R5 représentent un atome d'hydrogène ou de F, Cl, Br, I ou un groupe NO₂, OH, CN, alkyle en C₁-C₆, alcoxy en C₁-C₈, OCF₃, OCH₂CF₃, S-alkyle(C₁-C₄), COOH, HO-alkyle(C₁-C₄), alcoxy(C₁-C₄)-alkyle(C₁-C₄), (CH₂)ₒ-phényle, O-(CH₂)ₒ-phényle, o pouvant être 1-2, ou
R3 et R4 représentent ensemble -CH=CH-O-, -CH=CH-S, -O-(CH₂)ₚ-O-, où p = 1 ou 2, -O-CF₂-O-, -CH=CH-CH=CH- et
R5 représente un atome d'hydrogène.

5. Composition selon les revendications 1 à 4, dans laquelle R2 représente un atome d'hydrogène.

6. Composition selon les revendications 1 à 5, dans laquelle
R1 représente un atome d'hydrogène, un groupe CF₃, alkyle en C₁-C₄, phényle,
R2 représente un atome d'hydrogène,
A représente -CH₂-, -C₂H₄-, -C₃H₆-, -CH(OH)-, -(C=O)-, -CH=CH-, -CH=CH-CH₂-, -CO-CH₂-CH₂- ou -CO-NH-CH₂- ;
n est un nombre égal à 2 ou 3 ;
Cyc1 représente un cycle insaturé, 1 atome de carbone pouvant être remplacé par S ;
R3, R4, R5 représentent un atome d'hydrogène ou de F,
Cl, Br, I ou un groupe NO₂, OH, CN, alkyle en C₁-C₆, alcoxy en C₁-C₈, -O-CH₂-phényle, OCF₃, S-CH₃, COOH ou
R3 et R4 représentent ensemble -CH=CH-O-, -O-(CH₂)ₚ-O-, où p = 1 ou 2, -O-CF₂-O-, -CH=CH-CH=CH- et
R5 représente un atome d'hydrogène.

7. Composition selon les revendications 1 à 6, dans laquelle
A représente -CH₂- ou -CH₂-CH₂-.

8. Composition selon les revendications 1 à 7, dans laquelle
Cyc1 représente le groupe phényle.

9. Composition selon les revendications 1 à 7, dans laquelle
Cyc1 représente le groupe thiényle.

10. Composition selon les revendications 1 à 8, dans laquelle
Cyc1 est monosubstitué.

11. Composition selon la revendication 1, comprenant un composé choisi parmi le groupe : 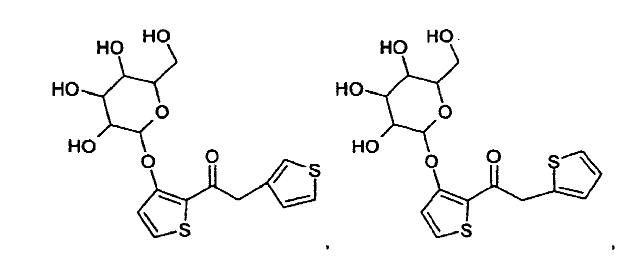 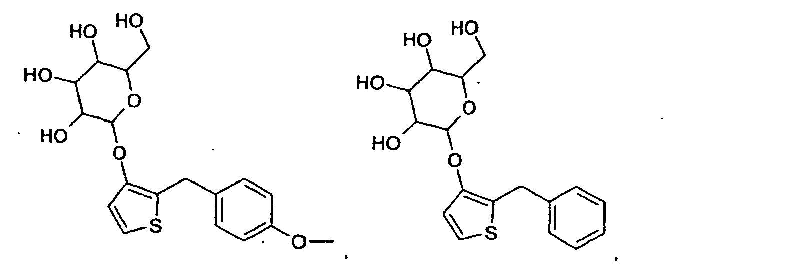 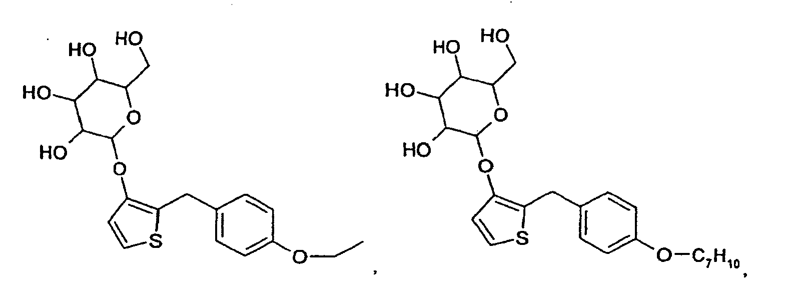 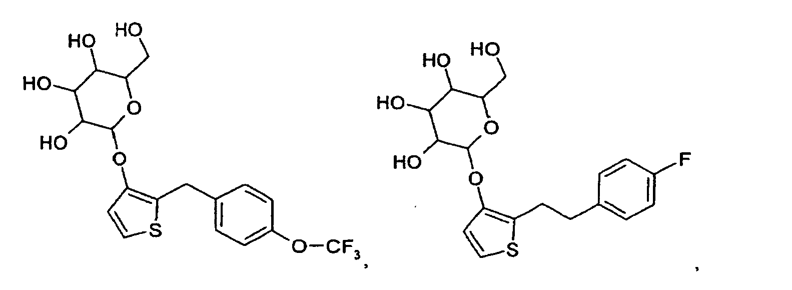 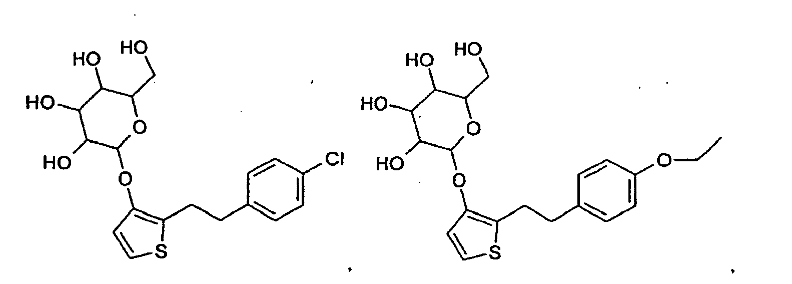 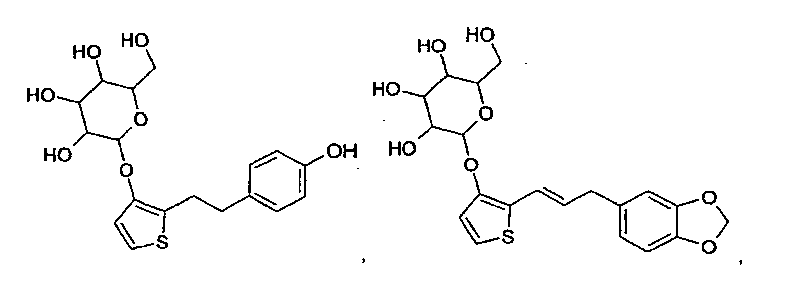 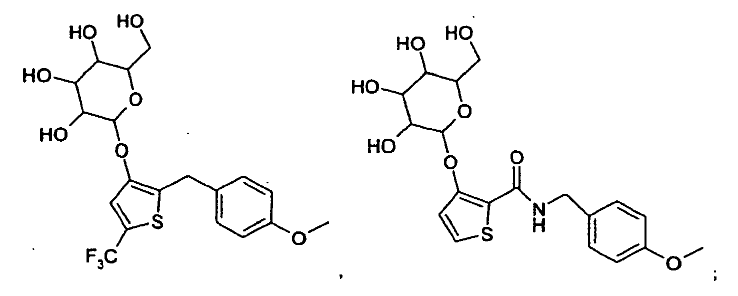 celui-ci dans la forme β-D-gluco.

12. Composition selon la revendication 1, comprenant un composé choisi parmi le groupe : 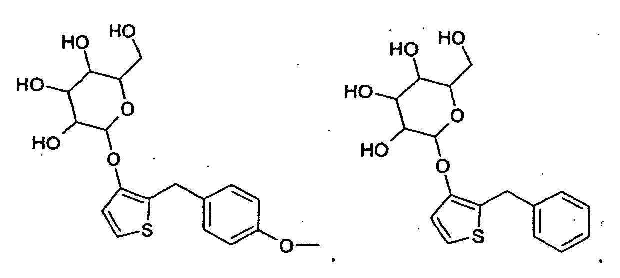 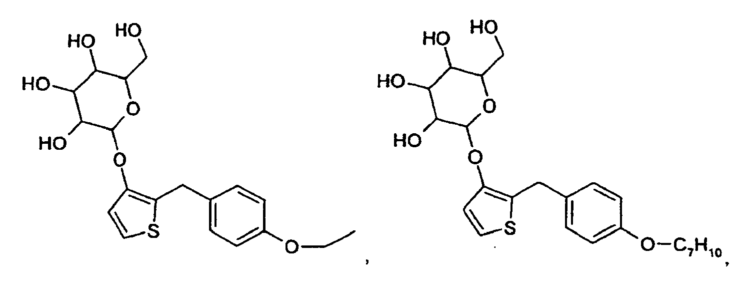 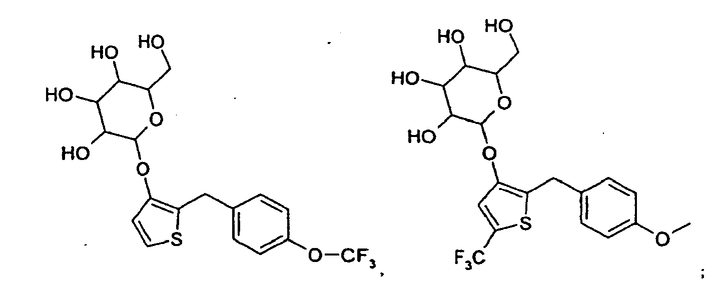 celui-ci dans la forme β-D-gluco.

13. Composition selon les revendications 1 à 12, **caractérisée en ce que** le biguanide est la metformine.

14. Composition selon les revendications 1 à 12, **caractérisée en ce que** la sulfonylurée est le tolbutamide, le glibenclamide, le glipizide ou le glimépiride.

15. Médicament contenant une composition selon une ou plusieurs des revendications 1 à 14.

16. Utilisation de la composition selon une ou plusieurs des revendications 1 à 14, pour la fabrication d'un médicament destiné au traitement du diabète du type 1 et du diabète du type 2.

17. Utilisation de la composition selon une ou plusieurs des revendications 1 à 14, pour la fabrication d'un médicament destiné à l'abaissement de la glycémie.

18. Utilisation d'un composé de formule I selon une ou plusieurs des revendications 1 à 12, en association avec la metformine, pour la fabrication d'un médicament destiné au traitement du diabète de type 1 et du diabète de type 2.

19. Utilisation d'un composé de formule I selon une ou plusieurs des revendications 1 à 12, en association avec la metformine, pour la fabrication d'un médicament destiné à l'abaissement de la glycémie.

20. Utilisation d'un composé de formule I selon l'une ou plusieurs des revendications 1 à 12 en association avec une sulfonylurée choisie parmi le groupe constitué par le tolbutamide, le glibenclamide, le glipizide ou le glimépiride, pour la fabrication d'un médicament destiné au traitement du diabète de type 1 et du diabète de type 2.

21. Utilisation d'un composé de formule I selon l'une ou plusieurs des revendications 1 à 12 en association avec une sulfonylurée choisie parmi le groupe constitué par le tolbutamide, le glibenclamide, le glipizide ou le glimépiride, pour la fabrication d'un médicament destiné à l'abaissement de la glycémie.

22. Utilisation d'un composé de formule I selon l'une ou plusieurs des revendications 1 à 12 en association avec l'insuline, pour la fabrication d'un médicament destiné au traitement du diabète de type 1 et du diabète de type 2.

23. Utilisation d'un composé de formule I selon l'une ou plusieurs des revendications 1 à 12 en association avec l'insuline, pour la fabrication d'un médicament destiné à l'abaissement de la glycémie.
